# EUROPEAN PATENT APPLICATION

(11) **EP 1 985 707 A1**
(43) Date of publication of application: **29.10.2008**
(21) Application number: 08007961.9
(22) Date of filing: 24.04.2008
(51) Int. Cl.: C12N 15/80, C12N 1/14, C12R 1/66

(54) **Method for the deletion of a large chromosomal region**

(30) Priority: 25.04.2007 JP 2007115013
(71) Applicant: NODA INSTITUTE FOR SCIENTIFIC RESEARCH, Noda-shi, Chiba-ken 278-0037 (JP)
(72) Inventor: Takahashi, Tadashi, Noda-shi, Chiba 278-0037 (JP); Koyama, Yasuji, Noda-shi, Chiba 278-0037 (JP)
(74) Representative: Strehl Schübel-Hopf & Partner

(57) **Abstract**

In order to efficiently delete a large chromosomal region with a length of from several tens to hundreds kb such as a cluster of biosynthesis genes encoding a toxic substance such as Aflatoxin, the present invention provides a method for the deletion of a large chromosomal region comprising transforming a transformant having an increased frequency of homologous recombination due to suppression of a *Ku gene,* which is a mitosporic filamentous fungus belonging to *Trichocomaceae* with a vector for the deletion of a chromosomal region, which comprises a pair of homologous region arms having a nucleotide sequence that is homologous with a fragment at either end of said chromosomal region, and deleting the chromosomal region by means of homologous recombination between the resulting transformant and said vector.

## Description

### Technical Field

The present invention relates to a method for the deletion of a large chromosomal region comprising transforming a transformant having an increased frequency of homologous recombination due to suppression of a *Ku gene,* which is a mitosporic filamentous fungus belonging to *Trichocomaceae,* for example, *Aspergillus* such as *Aspergillus sojae* and *Aspergillus oryzae* with a vector for the deletion of a chromosomal region, and deleting the chromosomal region by means of homologous recombination between the resulting transformant and said vector.

### Background of the Invention

*Aspergillus* strains such as *Aspergillus sojae* and *Aspergillus oryzae* have been industrially used in the production of brewed food such as soy sauce, sake (rice wine), soybean paste, etc. With a recent determination of the whole genomic sequence of *Aspergillus oryzae* and development of an exhaustive analysis of gene expression using a micro-array, it has been expected that genetic modification of their genes, especially their chromosomal modification would increase the productivity of an enzyme and improve a growth rate of these filamentous fungi.

However, as *Aspergillus* strains have a very low frequency of homologous recombination, it has been very difficult to produce a strain with a large area-deletion of an arbitrary chromosomal region by conventional methods. For example, as the frequency of homologous recombination is as low as 1~2 %, it will be necessary to prepare several tens to hundreds of transformants and select a desired strain from them in order to integrate even a single vector into an optional area of its chromosome (Non-Patent Document 1). Furthermore, it is necessary to select a recombinant strain having a recombination between both ends of an area to be deleted for obtaining the strain with a large-area deletion. But, it would be very difficult to select such recombinant for *Aspergillus* strains that have the very low frequency of homologous recombination. However, a recent study by the present inventors has revealed that the disruption of a gene involved in non-homologous recombination will significantly increase the frequency of gene targeting (homologous recombination) (Patent Document 1)..

By the way, Aflatoxin is a toxin produced by fungi belonging to *Aspergillus* such as *Aspergillus flavus* or *Aspergillus parasiticus,* and is known as the strongest cancer-causing agent among natural substances. There have been big problems about contamination of cereal due to these molds in tropical region. There was death en mass of turkeys in England in 1960, which was caused by uptake of the cereal contaminated by the mold (an accident of turkey's X disease). Since the above *Aspergillus* strains are closely-related to Aflatoxin-producing strains, they have been studied in a wide variety of views with suspicion of their Aflatoxin-producing capability (Non-Patent Document 2). As a result, it has been confirmed that although they will not produce Aflatoxin under any conditions, they have a homologue of a gene cluster of about 60 Kb for the Aflatoxin-biosynthesis of comprised the Aflatoxin-producing strains (Non-Patent Document 3).

It has been therefore strongly desired that the gene cluster should be removed from the *Aspergillus* strains for a further increase of safety of foods. However, it has been very difficult to prepare a mutant strain having deletion of the above Aflatoxin cluster since a set of genes in this cluster are not expressed (Non-Patent Document 4), making it impossible to carry out selection based on its phenotype in addition to the low frequency of their homologous recombination.
[Patent Document 1] JP 2006-158269
[Non-Patent Document 1] Takahashi et al., Mol. Gen. Genet. (2004) 272:344-52
[Non-Patent Document 2] Matsushima et al., Journal of the Japan Soy Sauce Research Institute, (2004) 31:5-11
[Non-Patent Document 3] Yu et al. (2004) Appl Environ Microbiol 70:1253-1262
[Non-Patent Document 4] Matsushima et al., Appl Microbiol Biothechnol 55: 771-776

### Disclosure of the Invention

### [Problems to be solved by the present invention]

The main purpose of the present invention is therefore to provide a method for efficiently deleting a large chromosomal region with a length of from several tens to hundreds kb such as a cluster of biosynthesis genes encoding a toxic substance such as Aflatoxin, and to a transformant produced by said method, which will not produce the toxic substance even after being manipulated with genetic engineering.

The present inventors succeeded in efficiently producing a strain having the deletion of such a large region as from ten-odd kb to about 200kb by using such *Aspergillus* strains with an increased frequency of homologous recombination as disclosed in Patent Document 1, and have completed the present invention.

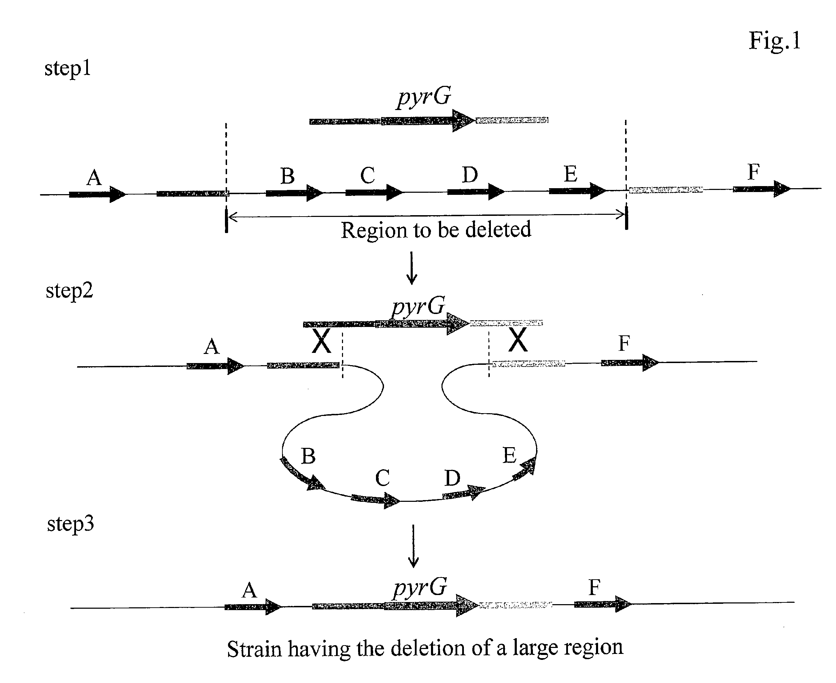

Thus, the present invention is related to a method for the deletion of a large chromosomal region comprising transforming a transformant having an increased frequency of homologous recombination due to suppression of a *Ku gene,* which is a mitosporic filamentous fungus belonging to *Trichocomaceae,* for example, *Aspergillus* such as *Aspergillus sojae* and *Aspergillus oryzae* with a vector for the deletion of a chromosomal region, which comprises a pair of homologous arms having a nucleotide sequence that is homologous with a fragment at either end of said chromosomal region, and deleting the chromosomal region by means of homologous recombination between the resulting transformant and said vector, and to a transformant produced by said method, which has a deleted region in chromosome. An example of the present method is illustrated in Fig.1.

According to the present invention, it is now possible to efficiently delete a large chromosomal region with a length of from several tens to hundreds kb such as a cluster of Aflatoxin-biosynthesis genes. As a result, it makes it easy to produce a strain having the deletion of a gene cluster occupying a large area in the chromosome, which is very preferable from safety point of view.

### Brief Description of Drawings

Fig.1 is a schematic figure showing one example of the method according to the present invention.
Fig.2 shows a summary and photographs in electrophoresis showing the results of PCR analysis of the genome of a strain having the deletion of a chromosomal region produced by the method according to present invention using *Aspergillus sojae* ASKUPTR8 strain.
Fig.3 shows a summary and photographs in electrophoresis showing the results of Southern hybridization of the genome of a strain having the deletion of a cluster (60 kb) of biosynthesis genes encoding Aflatoxin produced by the method according to present invention using *Aspergillus sojae* ASKUPTR8 strain.
Fig.4 shows a summary and photographs in electrophoresis showing the results of Southern hybridization of the genome of a strain having the deletion of a 190 kb region in the chromosome produced by the method according to present invention using *Aspergillus sojae* ASKUPTR8 strain.
Fig.5 shows a summary and photographs in electrophoresis showing the results of Southern hybridization of the genome of a strain having the deletion of a 200 kb region in the chromosome produced by the method according to present invention using *Aspergillus sojae* ASKUPTR8 strain.
Fig.6 shows a summary and photographs in electrophoresis showing the results of PCR analysis of the genome of a strain having the deletion of a chromosomal region produced by the method according to present invention using RkuN16ptr1 strain.
Fig.7 shows a summary and photographs in electrophoresis showing the results of Southern hybridization of the genome of a strain having the deletion of a cluster (60 kb) of biosynthesis genes encoding Aflatoxin produced by the method according to present invention using RkuN16ptr1 strain.

### Best Mode for Carrying Out the Present Invention

The "transformant having an increased frequency of homologous recombination due to suppression of a *Ku* gene, which is a mitosporic filamentous fungus belonging to *Trichocomaceae* " that was used in the present invention may be prepared by the method disclosed in Patent Document 1. Thus, the transformant can be prepared by suppressing the *Ku* gene of a mitosporic filamentous fungus belonging to *Trichocomaceae.* The scope and kinds of the fungus that may be mycologically classified into the "mitosporic filamentous fungus belonging to *Trichocomaceae"* are obvious for those skilled in the art. There may be mentioned as its representative examples strains belonging to *Aspergillus* such as *Aspergillus sojae* and *Aspergillus oryzae,* and strains belonging to *penicillium.* These strains may be commercially available from public depository authorities such as American Type Culture Collection (ATCC).

As already described in the above, the *Ku gene* such as *Ku70* and *Ku80* is a gene involved in the non-homologous recombination mechanism. Its representative examples include *Ku70* gene (SEQ ID NO.1) and *Ku80* gene (SEQ ID NO.2 or SEQ ID NO.3) derived from *Aspergillus sojae,* and *Ku70* gene (SEQ ID NO.4) and *Ku80* gene (SEQ ID NO.5 or SEQ ID NO.6) derived from *Aspergillus oryzae.* Homology (identity) in an amino acid level between the above *Ku70* genes and between the above *Ku80* genes was found to be as high as 95% or more. On the other hand, the homology in an amino acid level between these genes and homologues of *Neurospora crassa* is about 50%.

Accordingly, there may be mentioned as preferable examples of the *Ku gene,* a gene that encodes a protein consisting of an amino acid sequence represented by any one of SEQ ID Nos 1-6, or a protein consisting of the same amino acid sequence wherein one or several amino acid residues are replaced, deleted, or added, or a gene that encodes a protein having a high homology such as about 90% or more, preferably about 95% or more on a total average to the amino acid sequence represented by any one of SEQ ID Nos 1-6 and having a function relating to non-homologous recombination mechanism.

Furthermore, preferable examples of the *Ku gene* may include (a) DNA comprising a coding region represented by any one of SEQ ID Nos 1-6, or DNA being hybridized with DNA consisting a base sequence complementary with that of the DNA (a) under stringent conditions, and encoding a protein having the function relating to non-homologous recombination mechanism.

The coding region in any one of SEQ ID Nos 1-6 was determined based on the information about the genomic sequences of *Aspergillus sojae* and *Aspergillus oryzae*, comparison with the sequences of their homologues of *Neurospora crassa* and rules concerning an intron sequence such as GT-AG rule. The genomic sequence of the *Ku gene* of *Aspergillus sojae* was determined by amplifying a fragment in PCR using a genomic DNA of *Aspergillus sojae* ATCC46250 as a template and primers kuU459-KuL4222 and ku2U830-ku2L4937 (prepared based on the genomic sequences of *Aspergillus oryzae* and their homologues of *Neurospora crassa),* cloning the resulting fragment by means of TOPO-TA cloning kit (Invitrogen Co.) and subjecting it to a conventional sequence determination.

The hybridization may be performed in accordance with a method known in the art, for example, those described in Molecular cloning third. Ed. Cold Spring Harbor Lab. Press, 2001). When a commercially available library is used, the hybridization may be done according to instructions attached to it.

The hybridization may be performed in accordance with a method known in the art, for example, that described in Current protocols in molecular biology (edited by Frederick M. Ausubel et al., 1987). When a commercially available library is used, the hybridization may be done according to instructions attached to it.

The term "stringent conditions" means in this specification, for example, those of sodium concentration of 150~900mM, preferably 600~900mM, pH of 6~8 at 60°C~68°C.

The DNA that is hybridized with the DNA consisting of a base sequence complementary with that of the DNA comprising a coding region represented by any one of SEQ ID Nos 1-6 may include, for example, DNA consisting of a base sequence having identity (homology) with the whole base sequence of said DNA, of about 90% or more, preferably of about 95% or more on a total average. The identity between the base sequences may be determined by means of algorithm known to those skilled in the art, such as BLAST.

The suppression of the *Ku gene* in the transformant according to the present invention may be performed by any method known to those skilled in the art such as those actually described in the examples of the present specification. For example, the *Ku gene* may be disrupted by means of a *Ku* gene-disruption vector, or inactivated by means of antisense RNA method using a vector expressing an antisense of the *Ku gene.* The suppression of the *Ku gene* may be also attained by various methods based on RNA interference. The resulting transformant may have the frequency of homologous recombination that is increased by at least 10 times, preferably by at least 60 times.

Examples of the above transformants are *Aspergillus sojae* ASKUPTR8 strain (wh, *ΔpyrG,* Ku70::ptrA) and *Aspergillus oryzae* RkuN16ptr1 strain. The *Aspergillus sojae* ASKUPTR8 strain was deposited at the International Patent Organism Depository of National Institute of Advanced Industrial Science and Technology at AIST Tsukuba Central 6, 1-1, Higashi 1- chome Tsukuba-shi, lbaraki-ken 305-8566 Japan on December 2, 2004 with Accession No. FERM P-20311, and then transferred to international deposit under Budapest Treaty on November 17, 2005 with Accession No. FERM BP-10453.

The vector used for the deletion of a certain chromosomal region in the method according to the present invention is characterized by comprising a pair of homologous region arms having a nucleotide sequence that is homologous with a fragment at either end of said chromosomal region. The length of the homologous region arms may be optionally selected depending on the chromosomal region to be deled and the like, being preferably in a certain range, for example, about 1-3kb, in order to obtain a high frequency of homologous recombination. The phrase "nucleotide sequence that is homologous with a fragment at either end of said chromosomal region" means that a nucleotide sequence of said vector used for the deletion of a certain chromosomal region and that in the chromosome of the transformant have such a sufficient identity that homologous recombination will just occur between them. It is therefore not require that their nucleotide sequences are completely the same with each other.

When the chromosomal region to be deleted constitutes a cluster of biosynthesis genes encoding a toxic substance, the homologous region arms in the vector used for the deletion of a certain chromosomal region may have a nucleotide sequence of a gene or a part thereof that is located in the most marginal or peripheral part at either end of the region to be deleted. In such case, said nucleotide sequence may be amplified by any known DNA amplification technique such as PCR, and the homologous region arms may be then easily prepared by using the thus amplified sequence. Accordingly, in the case where the chromosomal region to be deleted is a cluster of Aflatoxin, a nucleotide sequence comprising moxY gene and pksA gene, respectively, may be used as the homologous region arm.

In the above case, as the nucleotide sequence of a gene or a part thereof that is located in the most marginal or peripheral part at both ends of the cluster of genes is comprised in the homologous region arms, said gene or a part thereof may be left even after the deletion of the subject chromosomal region. However, as the other genes in the cluster have been deleted, the toxic substance will never be produced any more. Thus, one of the purposes of the present invention, the provision of a transformant preferable in view of safety, is sufficiently fulfilled. Thus, all of the genes involved in biosynthesis of a substance are not necessarily comprised in the region (the cluster of genes) to be deleted in the method according to the present invention.

Alternatively, as long as the function of the gene that has been left after the deletion of the subject chromosomal region is not maintained any more, such as being incompetent regarding transcription, translation of a functional protein or peptide, due to the loss of a part of said gene, the advantages of the present invention will be attained. Thus, it is not necessary to completely delete the gene located at one of the both ends of the cluster of genes to be deleted.

Furthermore, in order to efficiently select the transformant having the deletion of a certain chromosomal region produced by homologous recombination according to the present method, it is preferable for any suitable selective marker gene known for those skilled in the art to be comprised between the pair of the homologous region arms of the vector for the deletion of a chromosomal region. The selective marker gene includes pyrG, sC, niaD and the like. As a result, said transformant having the deletion of a part of the chromosome, which is produced by the method according to the present invention, may be easily selected by using the above marker during a process of culture.

There is no limitation on size and location in the chromosome, etc. of the chromosomal region to be deleted. It is possible to delete a large chromosomal region with a length of from several tens to hundreds kb, for example 15 - 200kb by the present invention. A preferable example of such chromosomal region is the cluster of genes involved in biosynthesis of substances such as Aflatoxin, which should preferably not be produced by the strain from a safety aspect. The size and location in the chromosome of said genes as well as nucleotide sequences located in the most marginal or peripheral part at both ends of the region to be deleted are known for those skilled in the art.

The transformation with the vector for the deletion of a chromosomal region may be carried out by any method known for those skilled in the art, such as protoplast PEG method, electroporation and the like.

The present invention will be specifically explained below with reference to the examples, which should not be construed to limit the scope of the present invention. Unless otherwise described, the means and conditions for gene engineering techniques in the following examples are usual ones known to those skilled in the art, such as those described in Japanese Patent Publication No.1998-80196.

### Example

### Strains:

*Aspergillus sojae* ASKUPTR8 strain (wh, *ΔpyrG* Ku70::ptrA) and *Aspergillus oryzae* RkuN16ptr1 strain (wh, *ΔpyrG* Ku70::ptrA) were used. *Aspergillus sojae* ASKUPTR8 is a *Δ Ku70* (Ku70-disruption) strain prepared from *Aspergillus sojae* I-6 strain, which in turn is a *Δ pyrG* strain prepared from ATCC46250 (Takahashi et al.2004. *Aspergillus oryzae* RkuN16ptr1 strain is a *Δ Ku70* strain derived from RIB 40 strain according to the method disclosed in Patent Document 1.

### Culture medium:

Polypeptone dextrin (PD) medium (polypepton 1%, dextrin 2%, KH₂PO₄0.5%, NaNO₃ 0.1%, MgSO₄0.05%, casamino acid 0.1 %, pH 6.0), CzapekDox (CZ) minimum medium, and 1.2M sorbitol CZ (as regeneration medium) were used. CZ medium containing 2mg/ml 5fluoroortic acid (SIGMA) and 20mM Uridine was used for positive selection of a *pyrG*⁻ (Uridine-auxotrophy) strain.

### Transformation:

Conidium was inoculated on liquid PD medium (50 ml) containing 20 mM Uridine in a conical flask (150 ml) and subjected to shake culture for about 20 hours at 30°C, followed by collection of mycelium. The collected mycelium was washed with 0.7M KCI buffer, shaken gently in 0.7M KCI buffer containing 1% Lysing enzyme (Sigma Co.) for 3 hours at 30°C to prepare protoplast. The protoplast was washed with 1.2M sorbitol buffer, and tranformed by means of a protoplast PEG method. Regeneration of the resulting transformant was carried out on 1.2M sorbitol CZ medium containing 0.5% agar.

### Construction of a vector for the deletion of a large region

Primers used in the present examples are listed in Table 1 below.

**[Table 1]**

| Primers | Sequence (5' to 3') |
|---|---|
| A | CAATCTTCGTGCACATAAGTTCACGTT |
| B | GTTGAACATATCTGACAAGCCTTCCGAGCAGACGGTGCTAATCA |
| C | TGACTCGTTATTCTTTGCTCCATCGCGTCGTGGCGAATTCTCTCG |
| D | GGCGGTGCGCTGGCGGAAAAACGCAGA |
| E | TCCCCACCACACTGTAATTCATC |
| F | TGACTCGTTATTCTTTGCTCATAGCAGCTTCGGTTGTCTACTTT |
| G | TCCGCATTGGCTTAACCCAGAGT |
| H | TGACTCGTTATTCTTTGCTCATAGCCGGGATTTGTACCATTGGT |
| pksU | ACGGCTTCGGCGTTACCTCGTTCAACC |
| AF-L | CCCCCACATGCCGTTGACCTGGTCCTC |
| 190L | GTAGCAATGGTCATATAAACGAGCGA |
| 200L | TATACTCCGCTCTAACGTGCTGTGTG |
| PU | AGGCTTGTCAGATATGTTCAACG |
| PL | ATGAGCAAAGAATAACGAGTCAA |
| pkU | GGTGCACTGCGTGTCGTCCTGCAGACTACA |
| pkL | GTCGCCGCCGGATCTCATTGCAAAAGCTCT |
| moxU | GCACACCGGACGGAATTGAAATATCTC |
| moxL | GGCGGTGCGCTGGCGGAAAAACGCAGA |

### Construction of a vector for the deletion of a cluster (60 kb) of biosynthesis genes encoding Aflatoxin

A vector (pPkvb1) for the deletion of a cluster of biosynthesis genes encoding Aflatoxin was constructed by means of fusion PCR. It comprised a pair of arms of 2 kb each in both ends having pksA and moxY, respectively, and pyrG as a selective marker. Thus, the arm having pksA, the arm having moxA and the pyrG marker region were amplified by the 1^{st} PCR using the primers A-B, C-D and PU-PL, respectively, and *Aspergillus* genomic DNA as a template. The resulting amplified fragments were then purified with QlAquick Gel Extraction kit. The 2^{nd} PCR was carried out using the primers pksU-AF-L and the mixture of the above purified fragments as a template to obtain a vector (pPkv1) for the deletion of the cluster of biosynthesis genes encoding Aflatoxin.

### Construction of a vector for the deletion of a 190 kb region

A vector (pPk217-20k) for the deletion of a 190 kb region was constructed by means of fusion PCR. It comprised a pair of arms of 2 kb each in both ends, one of which having pksA and the other being located at 186 kb distance. Thus, the arm having pksA, the other arm and the pyrG marker region were amplified by the 1^{st} PCR using the primers A-B, E-F and PU-PL, respectively, and *Aspergillus* genomic DNA as a template. The resulting amplified fragments were then purified with QlAquick Gel Extraction kit. The 2^{nd} PCR was carried out using the primers pksU-190L and the mixture of the above purified fragments as a template to obtain a vector (pPk217-20k) for the deletion of the 190 kb region.

### Construction of a vector for the deletion of a 200 kb region

A vector (pPk217) for the deletion of a 200 kb region was constructed by means of fusion PCR. It comprised a pair of arms of 2 kb each in both ends, one of which having pksA and the other being located at 203 kb distance. Thus, the arm having pksA, the other arm and the pyrG marker region were amplified by the 1^{st} PCR using the primers A-B, G-H and PU-PL, respectively, and *Aspergillus* genomic DNA as a template. The resulting amplified fragments were then purified with QlAquick Gel Extraction kit. The 2^{nd} PCR was carried out using the primers pksU-200L and the mixture of the above-purified fragments as a template to obtain a vector (pPk217) for the deletion of the 200 kb region.]

### Conditions of fusion PCR

The fusion PCR was carried out in accordance with general procedures (Yang et al., (2004) Eukaryot Cell 3: 1359-1362). 1^{st} PCR: keeping for one min and 30 seconds at 94°C, followed by repeating 35 times a cycle of heating for 20 seconds at 94°C, for one second at 70°C, for 30 seconds at 55°C, and for 2 min. at 72°C; 2^{nd} PCR: keeping for one min and 30 seconds at 94°C, followed by repeating 35 times a cycle of heating for 20 seconds at 94°C, for one second at 70°C, for 30 seconds at 55°C, and for 5 min. and 30 seconds at 72°C. The amplification was done with KOD plus (TOYOBO)

### Southern hybridization

Southern hybridization was done in accordance with general procedure with a membrane filter of Hybond-N+ (Amersham Pharmacia Co.). Detection was made by means of DIG Luminescent Detection Kit (Roche) in accordance with a method recommended by the manufacturer. Probes for pksA and moxY were prepared by means of PCR DIG Probe Synthesis Kit (Roche) using the primers pkU-pkL and moxU-moxL, respectively.

### Results and Discussion

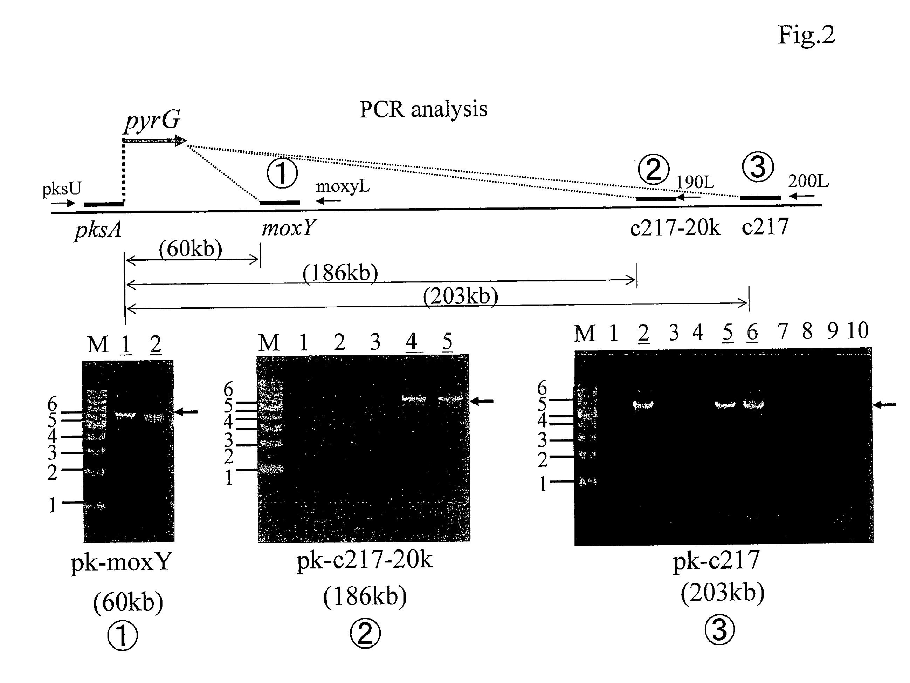

*Aspergillus sojae* ASKUPTR8 strain was then transformed with the vector (pPkvb1) for the deletion of a cluster of biosynthesis genes encoding Aflatoxin, the vector (pPk217-20k) for the deletion of a 190 kb region and the vector (pPk217) for the deletion of a 200 kb region, respectively. Genomic DNA was extracted from the resulting two transformants with the vector pPkvb1, the five transformants with the vector pPk217-20k and the ten transformants with the vector pPk217, and subjected to PCR and Southern hybridization for the purpose of confirmation of the integration of each vector. Thus, the PCR analysis revealed that a band was amplified with use of the primers pksU and AF-L (moxyL) amplified in the strains No.1 and No.2 having the deletion of the cluster of biosynthesis genes encoding Aflatoxin (Fig.2, ① lanes 1 and 2). Similarly, it was revealed that a band was amplified with use of the primers pksU and 190L amplified in the strains No.4 and No.5 having the deletion of the 190 kb region (Fig. 2, ②, lanes 4 and 5), and that a band was amplified with use of the primers pksU and 200L amplified in the strains No.5 and No.6 having the deletion of the 200 kb region (Fig. 2, ③, lanes 2, 5 and 6). On the other hand, no band was amplified with the genomic DNA from the strains having no deletion. Accordingly, it was estimated that the transformants with the above vectors also included a heterocaryon comprising both a nucleus having the target deletion and an original nucleus.

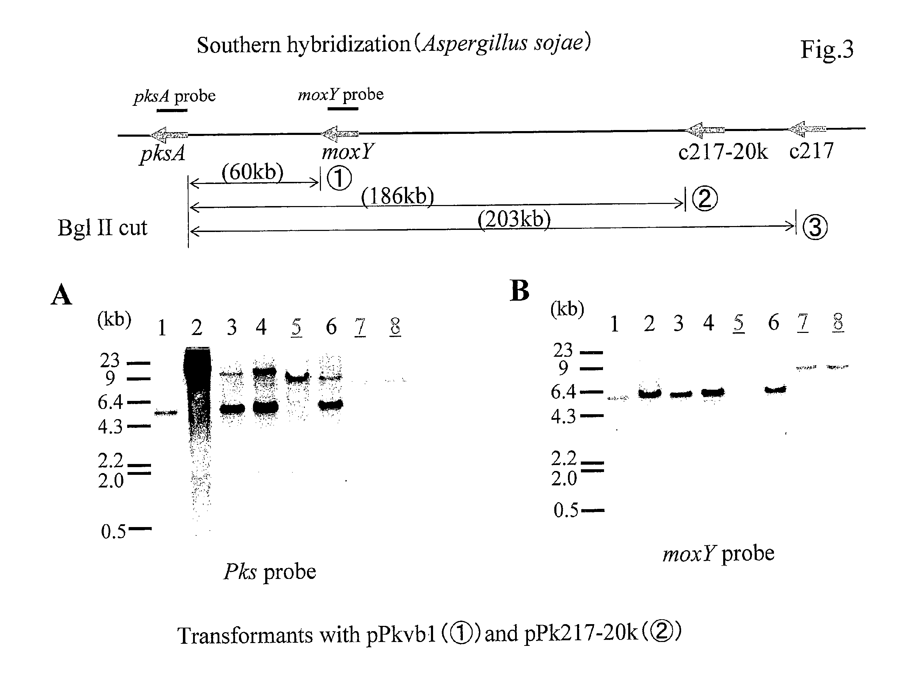

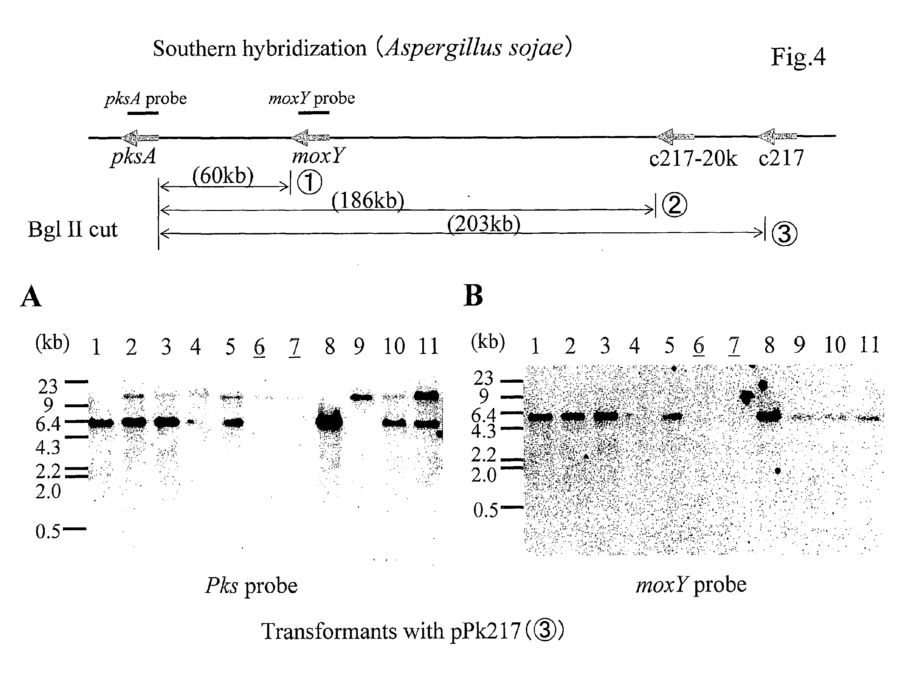

The Southern hybridization was carried out with genomic DNA of the transformants digested with *Bgl*II. It was observed that a band of 5.7 kb hybridized with the pksA probe in a parent strain (Fig.3A, lane 1) was shifted to 10 kb in the strains No.1 and No.2 having the deletion of the cluster of biosynthesis genes encoding Aflatoxin (Fig. 3A, lanes 7, 8) and to 15 kb in the strain No.4 having the deletion of the 190 kb region (Fig. 3A, lane 5). It was also observed that a band of 5.7 kb hybridized with the moxY probe in a parent strain (Fig.3 B, lane 1) was shifted to 10 kb in the strains No.1 and No.2 having the deletion of the cluster of biosynthesis genes encoding Aflatoxin while it was not found in the strain having the deletion of a 190 kb region (Fig. 3B, lane 5). Similarly, a band of 5.7 kb hybridized with the pksA probe in a parent strain (Fig.4A, lane 1) was shifted to 15 kb in the strains No.6 and No.7 having the deletion of the 200 kb region (Fig. 4A, lanes 6,7). It was also observed that a band of 5.7 kb hybridized with the moxY probe in a parent strain (Fig.4B, lane 1) was not found in the strains No.6 and No.7 having the deletion of a 200 kb region (Fig. 4B, lanes 6, 7). From these results, it was confirmed that the target region was finally deleted in the strains 1 and 2 having the deletion of the cluster of biosynthesis genes encoding Aflatoxin, the strain No.4 having the deletion of the 190 kb region and the strains No.6 and No.7 having the deletion of the 200 kb region. The frequency of obtaining the desired transformants was 100% (2 out of 2 strains) in the strains having the deletion of the cluster of biosynthesis genes encoding Aflatoxin, 20% (one out of 5 strains) in the strains having the deletion of 190 kb region, and 20% (2 out of 10 strains) in the strains having the deletion of 200 kb region.

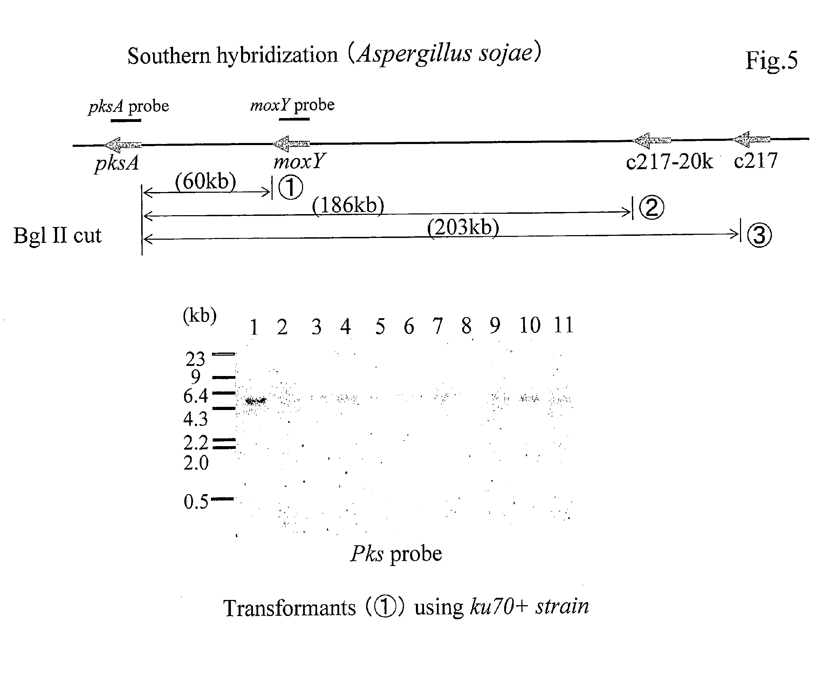

Next, preparation of a strain having the deletion of the cluster of biosynthesis genes encoding Aflatoxin was done using a strain wherein ku70 gene was not disrupted as a parent strain (*A*. *sojae* I-6) according to Non-Patent Document 1.
Thus, said strain was transformed with the vector (pPkvb1) for the deletion of a cluster of biosynthesis genes encoding Aflatoxin. Genomic DNA was extracted from the resulting ten transformants and subjected to the Southern hybridization as shown in Fig.5. There was no change in band pattern with the pksA probe in the parent strain (fig.5, lane 1) and the transformant (Fig.5, lanes 2-11). Thus, the strain having the deletion of the cluster of biosynthesis genes encoding Aflatoxin could not be obtained by using the strain wherein ku70 gene was not disrupted.

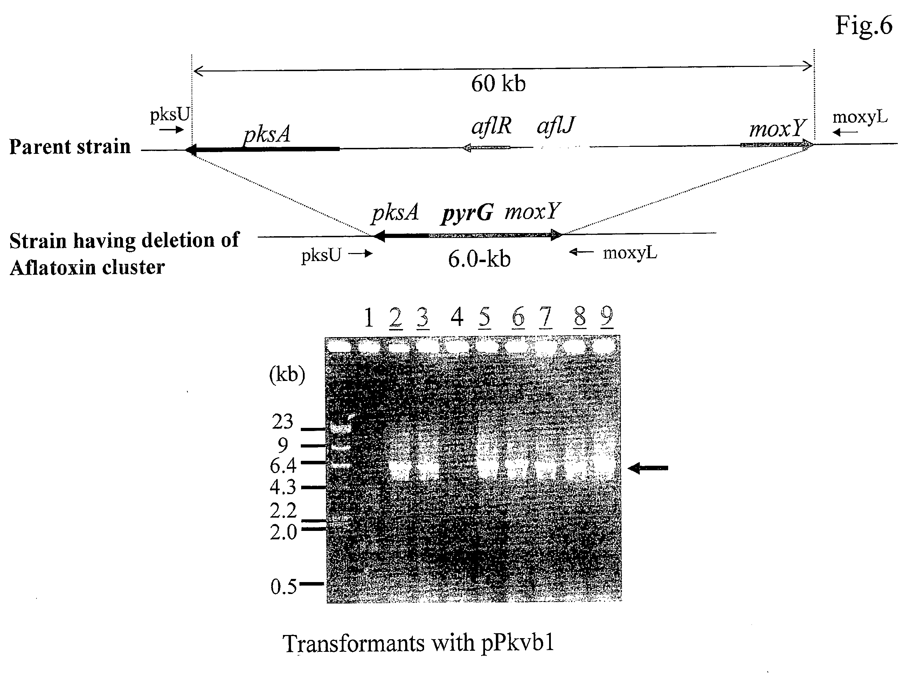

*A. oryzae* was similarly transformed with the vector pPkvb1 for the deletion of the cluster of biosynthesis genes encoding Aflatoxin. Genomic DNA was extracted from the resulting eight transformants obtained from *A. oryzae* RkuN16ptr1 (wh, *ΔpyrG, Ku70*::ptrA), and subjected to PCR using the primers pksU and moxyL. It was observed that a 6 kb band was amplified in 7 out of the 8 strains (Fig. 6, lanes 2, 3, 5-9) while no band was amplified in the parent strain. These results showed that there was at least one nucleus having the deletion of Aflatoxin cluster in the transformants wherein the 6 kb band was amplified.

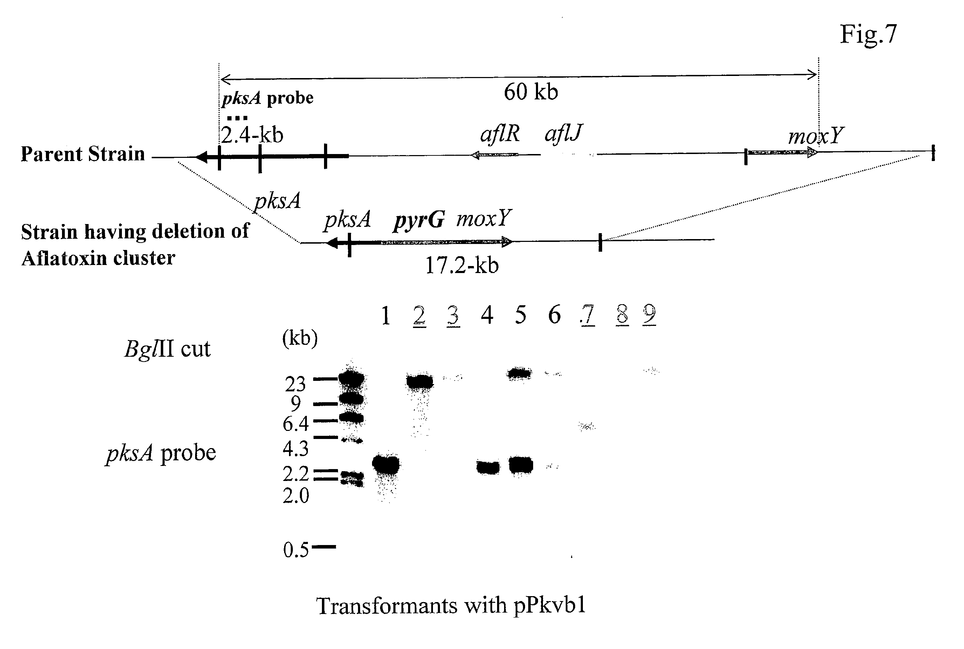

The Southern hybridization was carried out with genomic DNA of the transformants digested by *Bgl* II. It was observed that a band of 2.4 kb hybridized with the pksA probe in a parent strain (Fig.7, lane 1) was shifted in 5 out of the 8 strains (Fig.7, lanes 2, 3 7-9), showing the cluster of biosynthesis genes encoding Aflatoxin had been deleted in these transformants. As both 2.4 kb and 17.2 kb bands were observed in two out of 8 strains, these strains were confirmed to be a heterocaryon comprising both a nucleus having the deletion of the Aflatoxin cluster and an original nucleus (Fig.7, lanes 5, 6).

*A.oryzae* RIB40 *ΔpyrG* strain wherein *Ku70* was not disrupted was similarly transformed with said vector pPkvb1. The Southern hybridization analysis with respect to the resulting ten transformants revealed that only *Bgl* II band of 2.4 kb was observed in both each transformant and the parent strain, confirming that Aflatoxin genes had not been deleted.

The above frequencies were summarized in Table 2. It has been shown that a strain having the deletion of a large chromosomal region could be obtained with a high frequency by using the *Ku70*-disruption strains of *A.oryzae* and *A.sojae* while such strains having the deletion could not be obtained from a strain wherein *Ku70* was not disrupted. These results show that a strain having the deletion of a large region such as that of at least about 200kb, which has been very difficult to prepared, is now efficiently obtained by the present invention.

**[Table 2]**

| Strain | *A.sojae* | | | | *A.oryzae* | |
|---|---|---|---|---|---|---|
| Gene-type | *Δ ku70* | | | *ku70+* | *Δku70* | *ku70*+ |
| deleted region | 60kb | 100kb | 200kb | 60kb | 60kb | 60kb |
| Number of transformants | 2 | 5 | 10 | 10 | 8 | 10 |
| Number of transformant having the deletion | 2 | 1 | 2 | 0 | 5 | 0 |
| Frequencey (%) | 100 | 20 | 20 | 0 | 62.5 | 0 |

Additionally, preparation of a strain having the deletion of another large region was obtained, which region is not located in the vicinity of telomere of the chromosome No.3 where the cluster of biosynthesis genes encoding Aflatoxin are present. Thus, deletion was tried with respect to a region of about 15 kb comprising a tannase gene in the vicinity of centromere of the chromosome No.8 by using *Aspergillus oryzae* RkuN16ptr1 strain (wh, *ΔpyrG, Ku70*::ptrA). It was confirmed that the above deletion occurred in all of the resulting ten transformants. Accordingly it was shown that use of the *Ku70*-disruption strain enabled to efficiently obtain a strain having the deletion of a large region at any site in chromosome.

### [Industrial Applicability]

The present invention makes it possible not only to efficiently produce *Aspergillus* strains having the deletion of a certain region that should be preferably removed for increase of safety of foods such as the cluster of biosynthesis genes encoding Aflatoxin, but also to modify *Aspergillus* strains at a chromosomal level so as to be of great advantage in industrial fields.

SEQUENCE LISTING
<110> NODA INSTITUTE FOR SCIENTIFIC RESEARCH

<120> Method for the deletion of a large chromosomal region

<130> AB07025

<150> JP2007-115013
<151> 2007-04-25

<160> 6

<210> 1
<211> 2249
<212> DNA
<213> Aspergillus sojae

<220>
<223> Aspergillus sojae Ku70

<400> 1
ATG GCT GAC GAG GAT CAA TAT CGC GGA GAC GAC CAG ATC GAT GAG GAA 48
Met Ala Asp Glu Asp Gln Tyr Arg Gly Asp Asp Gln Ile Asp Glu Glu
1               5                   10                  15

GAG GAG GAG ATC GAC GAG AGT GTACACACTT TCAAACACAC CTGAAAGCTT CGGA 103
Glu Glu Glu Ile Asp Glu Ser
            20

GGCTAACATG TTATCAACCA AAATAG GGA TAC AAA ACA GTG AAA GAT GCC GTT 156
Gly Tyr Lys Thr Val Lys Asp Ala Val
    25                  30

CTT TTT GCT ATC GAA GTC AGC GAT TCG ATG CTC ACC CCT CGT CCA TCT 204
Leu Phe Ala Ile Glu Val Ser Asp Ser Met Leu Thr Pro Arg Pro Ser
        35                  40                  45

TCC GAT TCA AAG AAA CCT GCG GAG GAG TCC CCC ACA ACG GCG GCA CTA 252
Ser Asp Ser Lys Lys Pro Ala Glu Glu Ser Pro Thr Thr Ala Ala Leu
    50                  55                  60

AAA TGC GCA TAT CAT CTC ATG CAA CAA CGC ATT ATC TCT AAT CCC CGT 300
Lys Cys Ala Tyr His Leu Met Gln Gln Arg Ile Ile Ser Asn Pro Arg
65                  70                  75                  80

GAC ATG ATC GGT GTG CTA TTA TAT GGG ACG CAG GCG TCC AAA TTT TAT 348
Asp Met Ile Gly Val Leu Leu Tyr Gly Thr Gln Ala Ser Lys Phe Tyr
                85                  90                  95

GAC GAG GAT GAA AAC AGT CGA GGA GAC CTT TCA TAC CCA CAC TGC TAC 396
Asp Glu Asp Glu Asn Ser Arg Gly Asp Leu Ser Tyr Pro His Cys Tyr
            100                 105                 110

CTG TTC ACA GAC CTG GAT GTT CCC TCT GCG CAA GAA GTC AAG AAT CTT 444
Leu Phe Thr Asp Leu Asp Val Pro Ser Ala Gln Glu Val Lys Asn Leu
        115                 120                 125

CGG GCA CTG GCA CAA GAC GGC GAT GAA TCA GAG GAT GTA CTT AAG GCG 492
Arg Ala Leu Ala Gln Asp Gly Asp Glu Ser Glu Asp Val Leu Lys Ala
    130                 135                 140

TCA GGC GAG CGG GTC TCA ATG GCG AAC GTA CTC TTT TGC GCC AAT CAA 540
Ser Gly Glu Arg Val Ser Met Ala Asn Val Leu Phe Cys Ala Asn Gln
145                 150                 155                 160

ATA TTC ACG TCA AAA GCC CCC AAC TTC TTG TCT CGG CGA TTG TTC ATA 588
Ile Phe Thr Ser Lys Ala Pro Asn Phe Leu Ser Arg Arg Leu Phe Ile
                165                 170                 175

GTC ACC GAT AAT GAT GAC CCT CAT GGC GAT AAC AAA AGC TTG AGA TCC 636
Val Thr Asp Asn Asp Asp Pro His Gly Asp Asn Lys Ser Leu Arg Ser
            180                 185                 190

GCT GCA ACT GTA CGC GCG AAG GAC TTA TAC GAC CTC GGT GTC ACT ATT 684
Ala Ala Thr Val Arg Ala Lys Asp Leu Tyr Asp Leu Gly Val Thr Ile

        195                 200                 205

GAG CTG TTT CCG ATT TCT CGG CCA GAC CAT GAG TTC GAT ACC GCC AGG 732
Glu Leu Phe Pro Ile Ser Arg Pro Asp His Glu Phe Asp Thr Ala Arg
    210                 215                 220

TTC TAT GAC GTAAGATTA TATTGACTCA ATGTGAAGTA TCGCTGCTAA CAGCAATTAG 790
Phe Tyr Asp
225

GAT ATC ATC TAC AAG GCC TCT CCT TCG GAT CCA GAT GCC CCA GCG TAC 838
Asp Ile Ile Tyr Lys Ala Ser Pro Ser Asp Pro Asp Ala Pro Ala Tyr
        230                 235                 240

CTG CAA ACT GAT TCC AAG GCT TCT CCA GCT ACC GGG GAT GGG ATA TCA 886
Leu Gln Thr Asp Ser Lys Ala Ser Pro Ala Thr Gly Asp Gly Ile Ser
    245                 250                 255

CTG CTC AGT ACC CTC CTG TCC AGT ATC AAT TCG AGA TCT GTC CCA CGG 934
Leu Leu Ser Thr Leu Leu Ser Ser Ile Asn Ser Arg Ser Val Pro Arg
260                 265                 270                 275

CGT GCG CAG TTC TCC AAC ATA CCA TTG GAG CTG GGA CCA AAC TTC AAA 982
Arg Ala Gln Phe Ser Asn Ile Pro Leu Glu Leu Gly Pro Asn Phe Lys
                280                 285                 290

ATA TCT GTC TCG GGA TAT CTT TTG TTC AAG CGT CAA GCA CCT GCC AGA 1030
Ile Ser Val Ser Gly Tyr Leu Leu Phe Lys Arg Gln Ala Pro Ala Arg
            295                 300                 305

AAC TCC TTC ATC TGG CTC GGC GGT GAA CAG CCC CAG ATT GTC AAA GGA 1078
Asn Ser Phe Ile Trp Leu Gly Gly Glu Gln Pro Gln IleVal Lys Gly
310 315 320

GTG ACC ACT CAA ATC GCT GAC GAC ACG GCT CGC ACG ATT GAG AAG TGG 1126
Val Thr Thr Gln Ile Ala Asp Asp Thr Ala Arg Thr Ile Glu Lys Trp
325 330 335

GAA ATC AAG AAA GCT TAT AAG TTT GGG GGT GAT CAG GTT GCT TTC ACG 1174
Glu Ile Lys Lys Ala Tyr Lys Phe Gly Gly Asp Gln Val Ala Phe Thr
340 345 350 355

CCC GAA GAG ATG AAG TCA CTG AGG AAC TTC GGT GAT CCT GTC ATC CGT 1222
Pro Glu Glu Met Lys Ser Leu Arg Asn Phe Gly Asp Pro Val Ile Arg
360 365 370

ATA ATT GGG TTC AAG CCC CTT TCT GCA CTT CCG TTC TGG GCC AAT ATC 1270
Ile Ile Gly Phe Lys Pro Leu Ser Ala Leu Pro Phe Trp Ala Asn Ile
375 380 385

AAA CAC CCT TCC TTC ATA TAC CCA TCG GAA GAA GAT TTC GTG GGC TCC 1318
Lys His Pro Ser Phe Ile Tyr Pro Ser Glu Glu Asp Phe Val Gly Ser
390 395 400

ACG CGA GTC TTT TCT GCT TTG CAT CAG ACA CTT CTC CGG GAT AAA AAG 1366
Thr Arg Val Phe Ser Ala Leu His Gln Thr Leu Leu Arg Asp Lys Lys
405 410 415

GCC GCA CTT GTC TGG TTC ATT GCG CGT AAA AAC GCA AGC CCT GTT CTG 1414
Ala Ala Leu Val Trp Phe Ile Ala Arg Lys Asn Ala Ser Pro Val Leu
420 425 430 435

GGG GCT ATG GTC GCT GGC GAA GAG AAA CTA GAC GAG AGT GGC GTC CAG 1462
Gly Ala Met Val Ala Gly Glu Glu Lys Leu Asp Glu Ser Gly Val Gln
440 445 450

AAG TTT CCT CCA GGA ATG TGG ATA ATA CCT CTC CCG TTC GCT GAT GAC 1510
Lys Phe Pro Pro Gly Met Trp Ile Ile Pro Leu Pro Phe Ala Asp Asp
455 460 465

GTC CGT CAA AAC CCT GAA ACC ACA CTC CAT GTT GCA CCC GAG CCA TTG 1558
Val Arg Gln Asn Pro Glu Thr Thr Leu His Val Ala Pro Glu Pro Leu
470 475 480

ATC GAT CAA ATG CGG TAT ATT GTT CAG CAA CTG CAA CTT CCA AAG GCG 1606
Ile Asp Gln Met Arg Tyr Ile Val Gln Gln Leu Gln Leu Pro Lys Ala

485                490                495

TCT TAC GAC CCC TTC AAG TAC CCT AAT CCA T GTAAGCTTCT GCCAACTTCC          1657
Ser Tyr Asp Pro Phe Lys Tyr Pro Asn Pro S
500                505                510

TGCACAGAAA  CTCTGGCATT  AACCTATTGC TCTGTTAG  CC CTC CAA TGG CAT          1709
                                              er Leu Gln Trp His

TAT CGC ATT CTA CAA GCC TTA GCG TTA GAT GAG GAC CTC CCG GAG AAG          1757
Tyr Arg Ile Leu Gln Ala Leu Ala Leu Asp Glu Asp Leu Pro Glu Lys
515                520                525                530

CCA GAA GAC AAA ACG TTG CCC AGA TAT CGG CAG ATT GAT AAA GTATATC          1806
Pro Glu Asp Lys Thr Leu Pro Arg Tyr Arg Gln Ile Asp Lys
                535                540

ACACATTCCT  ATTCTTTCCA  CGGATCTTGC  TGACCTTCGC TTAG CGC ACT GGC          1859
                                                    Arg Thr Gly
                                                    545

GAC TAT GTA TTG TCT TGG GCC GAC GAG TTG GAA AAG CAA TAC GCG AAA          1907
Asp Tyr Val Leu Ser Trp Ala Asp Glu Leu Glu Lys Gln Tyr Ala Lys
        550                555                560

ATA TCG GCA CAT GGC CCG AAG AGC ACA CTC GTC GAA CGA AGC GCC AAA          1955
Ile Ser Ala His Gly Pro Lys Ser Thr Leu Val Glu Arg Ser Ala Lys
565                570                575

GAC CGA ACA TCT GAA GTT GAG GAT GCA GCC CCG AAG CCA TAC AAG AAA          2003
Asp Arg Thr Ser Glu Val Glu Asp Ala Ala Pro Lys Pro Tyr Lys Lys
580                585                590                595

GTG AAG GTG GAG ACA GAC GAG CAA GGT GTT GAA GAT GTA GTG CGA GCC          2051
Val Lys Val Glu Thr Asp Glu Gln Gly Val Glu Asp Val Val Arg Ala
                600                605                610

CCT TAC CAA AAG GGA TCG CTA TCG AAG GTGACTATTA CCTGCCCCCT AGGCT          2103
Pro Tyr Gln Lys Gly Ser Leu Ser Lys
                615                620

TTAATTTGGA CTAACTAACG  CGCGTGACTT GTGTGTAG  CTT ACT GTA CCC GTC          2156
                                            Leu Thr Val Pro Val
                                                            625

CTC AAA AAC TTC CTG AAA GCC CAT GGA CGC TCC GCT GCT GGG AAG AAA          2204
Leu Lys Asn Phe Leu Lys Ala His Gly Arg Ser Ala Ala Gly Lys Lys
                630                635                640

AAA GAA CTC GTT GAG CGT GTG GAG GAG TAC CTG GAG CAG AAG TGA              2249
Lys Glu Leu Val Glu Arg Val Glu Glu Tyr Leu Glu Gln Lys Sto
                645                650                655

<210> 2
<211> 2707
<212> DNA
<213> Aspergillus sojae

<220>
<223> Aspergillus sojae Ku80 Long

<400> 2
ATG GCG GAC AAG GAA GCA ACT GTG TAT ATT GTG GAC GTT GGG AGG TCC            48
Met Ala Asp Lys Glu Ala Thr Val Tyr Ile Val Asp Val Gly Arg Ser
  1                5                10                15

ATG GGA GAA TGT CGC AAT GGC CGA TCA GTG ACT GAT CTT GAA TGG GCC            96
Met Gly Glu Cys Arg Asn Gly Arg Ser Val Thr Asp Leu Glu Trp Ala
                20                25                30

ATG CAG TAT GTC TGG GAT CGC ATT ACA GGA ACA GTGAGTGGCA GTCGTCACAA         149
Met Gln Tyr Val Trp Asp Arg Ile Thr Gly Thr
        35                40

TTGGGCTGCA TTCGTTAAAT ATCTTGCTCA ATTTCAGACC AG GTG GCC ACT GGC 203
Val Ala Thr Gly
    45

CGT AAA ACT GCC ACG ATG GGT GTG ATC GGA CTC AGG ACA GAT GGTATGTA 253
Arg Lys Thr Ala Thr Met Gly Val Ile Gly Leu Arg Thr Asp G
50 55 60

TACTTCTGAA TACTGTATGC GGTTCATACA CTGAACCAAA AAAATTAG AA ACG TCC AAC 312
lu Thr Ser Asn
65

GAA CTT GAA GAT GAC GTA CAT TTC TCT CAC ATT GCA GTT CTG TCG AAC 360
Glu Leu Glu Asp Asp Val His Phe Ser His Ile Ala Val Leu Ser Asn
70 75 80

ATC AAA CA GTATGCTTTC CACTCTATGA TAATTTGGTT CGTGCGCCAA ACTGACGAGG 418
Ile Lys Gl

ACGTCAAG G TTT CTT ATG CCG GAC ATT CGG AAA CTG GAA GAT GAA TTG 466
n Phe Leu Met Pro Asp Ile Arg Lys Leu Glu Asp Glu Leu
85 90 95

AAA CCG AGC AAA ACG GAC AAG GGA GAC G GTAAGTTTTT TGTAAGCCAC TAGG 518
Lys Pro Ser Lys Thr Asp Lys Gly Asp A
100 105

ACCTAC TGTCCACTTA CTAAACTTCA TTCTCTAG CT ATT TCC GCT ATT ATC TTG 572
la Ile Ser Ala Ile Ile Leu
110

GCT ATT CAG ATG ATT ATC ACG CAT TGC AAG AAG TTG AAG TAC AGG CGC 620
Ala Ile Gln Met Ile Ile Thr His Cys Lys Lys Leu Lys Tyr Arg Arg
115 120 125

AAG ATC GCC CTA GTC ACT AAC GGA CAG GGG CGC ATG AGT GAT GAG GAC 668
Lys Ile Ala Leu Val Thr Asn Gly Gln Gly Arg Met Ser Asp Glu Asp
130 135 140 145

CTG GGC GAG ATT GTG AAA AAG GTC AAG GAA GAT AAC ATC GAG CTT GTT 716
Leu Gly Glu Ile Val Lys Lys Val Lys Glu Asp Asn Ile Glu Leu Val
150 155 160

GTT AT GTCAGTGATT TGCTACAAGA TAGCAACGAAACAAAAAGCTAACGTCAAG CAG G 774
Val Me t

GGA ATT GAT TTC GAT GAC CCT GAG TAC GGT TAC AAA GAA GAA GAC AAA 822
Gly Ile Asp Phe Asp Asp Pro Glu Tyr Gly Tyr Lys Glu Glu Asp Lys
165 170 175

GAC CCT CGC AAG GTAGCGATAT CTCTTGCGCA GCTTTATTCG TATCTAATAA CTAAA 879
Asp Pro Arg Lys
180

ACAG GCC GAA AAC GAA ACT CTC TTG CGT ACC CTC GTG GAA GAT TGT GAT 928
Ala Glu Asn Glu Thr Leu Leu Arg Thr Leu Val Glu Asp Cys Asp
185 190 195

GGA GTT TAT GGA ACA TTC GAG CAG GCT GTG GCT GAA CTA GAC ATT CCC 976
Gly Val Tyr Gly Thr Phe Glu Gln Ala Val Ala Glu Leu Asp Ile Pro
200 205 210

CGT GTC AAG TCT GTC AGG TCA GTG GCA AGC TTT AAA GGA TAT CTC CAA 1024
Arg Val Lys Ser Val Arg Ser Val Ala Ser Phe Lys Gly Tyr Leu Gln
215 220 225 230

CTA GGC AAC CCA GAG GAT TAT GAC TCT GCT CTC CGC ATT CCT GTT GAA 1072
Leu Gly Asn Pro Glu Asp Tyr Asp Ser Ala Leu Arg Ile Pro Val Glu
235 240 245

AGG TAC TAC CGG ACT TAC CCG GCC AAA CCC CCA ACC GCA AGT TCT TTC 1120
Arg Tyr Tyr Arg Thr Tyr Pro Ala Lys Pro Pro Thr Ala Ser Ser Phe
250 255 260

GTC CTG CGC TCA GAG CCT GAA GCT GGA CAA GAA GAG GCA GAG TCA TCT 1168
Val Leu Arg Ser Glu Pro Glu Ala Gly Gln Glu Glu Ala Glu Ser Ser
265 270 275

GAG GCT GCT GCT GCT ACA CAA AAA GGG AGC CAG TCT GGA GAT ATC GGA 1216
Glu Ala Ala Ala Ala Thr Gln Lys Gly Ser Gln Ser Gly Asp Ile Gly
280 285 290

CTC ACT ACT GTG AGA ACC ATG AGA ACA TAT CAA GTT GAG GAC AAA AGT 1264
Leu Thr Thr Val Arg Thr Met Arg Thr Tyr Gln Val Glu Asp Lys Ser
295 300 305 310

GCA CCG GGT GGG AAA ATC GAC ATC GAA CGA GAT GAC CTC GCC AAA GGA 1312
Ala Pro Gly Gly Lys Ile Asp Ile Glu Arg Asp Asp Leu Ala Lys Gly
315 320 325

TAT GAG TAT GGA CGG ACA GCA GTT CAC ATT AGT GAA ACC GAC GAG AAC 1360
Tyr Glu Tyr Gly Arg Thr Ala Val His Ile Ser Glu Thr Asp Glu Asn
330 335 340

ATC ACG ATT CTC GAT ACA TTC GCA GGG CTG GAG TTG ATG GGC TTC ATC 1408
Ile Thr Ile Leu Asp Thr Phe Ala Gly Leu Glu Leu Met Gly Phe Ile
345 350 355

CAG ACT GAC CGG GTATGTCTTG CTGAAGTCGC CTCGGTGCAT GCTCTGACAC GATTAAT 1467
Gln Thr Asp Arg
360

TATAG TAT CAA CGT TAT ATG CAC ATG TCC AAC ACA AAC ATC ATA ATT GCA 1517
Tyr Gln Arg Tyr Met His Met Ser Asn Thr Asn Ile Ile Ile Ala
365 370 375

CAA CGT GCC AAC GAC AAA GCA GCC CTT GCC CTT TCA TCC TTT ATA CAC 1565
Gln Arg Ala Asn Asp Lys Ala Ala Leu Ala Leu Ser Ser Phe Ile His
380 385 390

GCG CTT TTT GAG CTA GAA TGC TAT GCT GTT GCT CGC CTA GTC GTG AAA 1613
Ala Leu Phe Glu Leu Glu Cys Tyr Ala Val Ala Arg Leu Val Val Lys
395 400 405

GAG AAC AAG CCA CCT GTT ATA GTC TTG CTC GCG CCC TCG ATC GAG CCT 1661
Glu Asn Lys Pro Pro Val Ile Val Leu Leu Ala Pro Ser Ile Glu Pro
410 415 420 425

GAT TAT GAA TGC CTT CTC GAA GTC CAG TTA CCA TTC GCG GAA GAT GTC 1709
Asp Tyr Glu Cys Leu Leu Glu Val Gln Leu Pro Phe Ala Glu Asp Val
430 435 440

CGA ACC TAT CGG TTC CCT CCT CTG GAT AAA GTG ATT ACT GTT TCT GGA 1757
Arg Thr Tyr Arg Phe Pro Pro Leu Asp Lys Val Ile Thr Val Ser Gly
445 450 455

AAG GTT GTG ACG CAA CAC CGG AAT CTT CCC AAT GAT GAT TTA CTC GAT 1805
Lys Val Val Thr Gln His Arg Asn Leu Pro Asn Asp Asp Leu Leu Asp
460 465 470

GTG ATG GGC AAG TAC GTG AAT AGT ATG GAG CTT GTC GAC GCA GAT GAG 1853
Val Met Gly Lys Tyr Val Asn Ser Met Glu Leu Val Asp Ala Asp Glu
475 480 485

GAT GG GTAGGTTTAT GCCTAAAAGA TTCCGAATCT CTTCTCATTG ACAAAACCAG G 1909
Asp Gl y
490

GAT CCA GTT GAG ACT TTC CCT ATC GAC GAC TCG TAT TCG CCT GTT TTG 1957
Asp Pro Val Glu Thr Phe Pro Ile Asp Asp Ser Tyr Ser Pro Val Leu
495 500 505

CAC CGG ATT GAC GCC GCC ATC CGT GCT CGG GCT ATA CAC CCT GAC CAG 2005
His Arg Ile Asp Ala Ala Ile Arg Ala Arg Ala Ile His Pro Asp Gln
510 515 520

CCC ATA CCT CCT CCA TCA GAG AGA CTG ACA AAA TTC TCA CAC CCA CGA 2053
Pro Ile Pro Pro Pro Ser Glu Arg Leu Thr Lys Phe Ser His Pro Arg
525 530 535

GAG GAT CTC ATC GAG AGA TCA CAG AAA TAC CTA GAG AAG TTG ATC GAG 2101
Glu Asp Leu Ile Glu Arg Ser Gln Lys Tyr Leu Glu Lys Leu Ile Glu
540 545 550 555

ATA GCC GAT GTT AAG AAG G GTTGGACATC ATCCCACAAT CAAGTCTATC AGGCTG 2156
Ile Ala Asp Val Lys Lys V
560

CTAATTCTGT TACCAG TT CCT CCC AAA GCG AAG GGT CGT AAG CGC ACT CGC 2207
al Pro Pro Lys Ala Lys Gly Arg Lys Arg Thr Arg
565 570

GAA ACT GAG AAG CCG CTT TCC GGA CTC GAC GTC GAT GCC CTG CTT CAT 2255
Glu Thr Glu Lys Pro Leu Ser Gly Leu Asp Val Asp Ala Leu Leu His
575 580 585

CAT GAA AAG CGC GCC AAG ATA TCT CCC AAC AAT GCC ATT CCC GAG TTC 2303
His Glu Lys Arg Ala Lys Ile Ser Pro Asn Asn Ala Ile Pro Glu Phe
590 595 600 605

AAG CAG ACT CTC GCA CAG GCC GAG AAT ATC GAG GCC ATC AAA GAC GCT 2351
Lys Gln Thr Leu Ala Gln Ala Glu Asn Ile Glu Ala Ile Lys Asp Ala
610 615 620

ACA AAG CAG ATG ATG GTT ATC GTT GAA GAT CAA ATC AAA CAC AGT CTC 2399
Thr Lys Gln Met Met Val Ile Val Glu Asp Gln Ile Lys His Ser Leu
625 630 635

GGT GAT GCT AAC TAC GAC CGG GTC ATT GAA GCG CTG GGC ACG ATG CGT 2447
Gly Asp Ala Asn Tyr Asp Arg Val Ile Glu Ala Leu Gly Thr Met Arg
640 645 650

GAC GAG TTG GTA TCA TAC GAA GAG CCT ACC TCC TAC AAT AAC TTC CTA 2495
Asp Glu Leu Val Ser Tyr Glu Glu Pro Thr Ser Tyr Asn Asn Phe Leu
655 660 665

GGC CAG CTC AAG GAT AAG TTG TTA CAG GAG AAG CTT GGA GGA GAT CGA 2543
Gly Gln Leu Lys Asp Lys Leu Leu Gln Glu Lys Leu Gly Gly Asp Arg
670 675 680 685

CAA GAG CTG TGG TGG CTT ATT CGA CGA AAC AAG CTG GGA CTT GTC ACT 2591
Gln Glu Leu Trp Trp Leu Ile Arg Arg Asn Lys Leu Gly Leu Val Thr
690 695 700

CAG CGC GAG TCG GAT CAA TCT AGG GTT ACC GAT ACG GAA GCC AAA GAA G 2640
Gln Arg Glu Ser Asp Gln Ser Arg Val Thr Asp Thr Glu Ala Lys Glu
705 710 715

TAAGTCTCAT TAAGATGAAG GAGTGACCTA GACTAACCGC AGTTTACAG TTC ATG TCC 2698
Phe Met Ser
720

GCC AAA TGA 2707
Ala Lys Stop

<210> 3
<211> 2666
<212> DNA
<213> Aspergillus sojae

<220>
<223> Aspergillus sojae Ku80 Short

<400> 3
ATG GCG GAC AAG GAA GCA ACT GTG TAT ATT GTG GAC GTT GGG AGG TCC 48
Met Ala Asp Lys Glu Ala Thr Val Tyr Ile Val Asp Val Gly Arg Ser
1               5                   10                  15

ATG GGA GAA TGT CGC AAT GGC CGA TCA GTG ACT GAT CTT GAA TGG GCC 96
Met Gly Glu Cys Arg Asn Gly Arg Ser Val Thr Asp Leu Glu Trp Ala
            20                  25                  30

ATG CAG TAT GTC TGG GAT CGC ATT ACA GGA ACA GTGAGTGGCA GTCGTCACAA 149
Met Gln Tyr Val Trp Asp Arg Ile Thr Gly Thr
        35                  40

TTGGGCTGCA TTCGTTAAAT ATCTTGCTCA ATTTCAGACC AG GTG GCC ACT GGC 203
Val Ala Thr Gly
    45

CGT AAA ACT GCC ACG ATG GGT GTG ATC GGA CTC AGG ACA GAT GGTATGTA 253
Arg Lys Thr Ala Thr Met Gly Val Ile Gly Leu Arg Thr Asp G
50 55 60

TACTTCTGAA TACTGTATGC GGTTCATACA CTGAACCAAA AAAATTAG AA ACG TCC AAC 312
lu Thr Ser Asn
65

GAA CTT GAA GAT GAC GTA CAT TTC TCT CAC ATT GCA GTT CTG TCG AAC 360
Glu Leu Glu Asp Asp Val His Phe Ser His Ile Ala Val Leu Ser Asn
70 75 80

ATC AAA CA GTATGCTTTC CACTCTATGA TAATTTGGTT CGTGCGCCAA ACTGACGAGG 418
Ile Lys Gl

ACGTCAAG G TTT CTT ATG CCG GAC ATT CGG AAA CTG GAA GAT GAA TTG 466
n Phe Leu Met Pro Asp Ile Arg Lys Leu Glu Asp Glu Leu
85 90 95

AAA CCG AGC AAA ACG GAC AAG GGA GAC G GTAAGTTTTT TGTAAGCCAC TAGG 518
Lys Pro Ser Lys Thr Asp Lys Gly Asp A
100 105

ACCTAC TGTCCACTTA CTAAACTTCA TTCTCTAG CT ATT TCC GCT ATT ATC TTG 572
la Ile Ser Ala Ile Ile Leu
110

GCT ATT CAG ATG ATT ATC ACG CAT TGC AAG AAG TTG AAG TAC AGG CGC 620
Ala Ile Gln Met Ile Ile Thr His Cys Lys Lys Leu Lys Tyr Arg Arg
115 120 125

AAG ATC GCC CTA GTC ACT AAC GGA CAG GGG CGC ATG AGT GAT GAG GAC 668
Lys Ile Ala Leu Val Thr Asn Gly Gln Gly Arg Met Ser Asp Glu Asp
130 135 140 145

CTG GGC GAG ATT GTG AAA AAG GTC AAG GAA GAT AAC ATC GAG CTT GTT 716
Leu Gly Glu Ile Val Lys Lys Val Lys Glu Asp Asn Ile Glu Leu Val
150 155 160

GTT AT GTCAGTGATT TGCTACAAGA TAGCAACGAAACAAAAAGCTAACGTCAAG CAG G 774
Val Me t

GGA ATT GAT TTC GAT GAC CCT GAG TAC GGT TAC AAA GAA GAA GAC AAA 822
Gly Ile Asp Phe Asp Asp Pro Glu Tyr Gly Tyr Lys Glu Glu Asp Lys
165 170 175

GAC CCT CGC AAG GTAGCGATAT CTCTTGCGCA GCTTTATTCG TATCTAATAA CTAAA 879
Asp Pro Arg Lys
180

ACAG GCC GAA AAC GAA ACT CTC TTG CGT ACC CTC GTG GAA GAT TGT GAT 928
Ala Glu Asn Glu Thr Leu Leu Arg Thr Leu Val Glu Asp Cys Asp
185 190 195

GGA GTT TAT GGA ACA TTC GAG CAG GCT GTG GCT GAA CTA GAC ATT CCC 976
Gly Val Tyr Gly Thr Phe Glu Gln Ala Val Ala Glu Leu Asp Ile Pro
200 205 210

CGT GTC AAG TCT GTC AGG TCA GTG GCA AGC TTT AAA GGA TAT CTC CAA 1024
Arg Val Lys Ser Val Arg Ser Val Ala Ser Phe Lys Gly Tyr Leu Gln
215 220 225 230

CTA GGC AAC CCA GAG GAT TAT GAC TCT GCT CTC CGC ATT CCT GTT GAA 1072
Leu Gly Asn Pro Glu Asp Tyr Asp Ser Ala Leu Arg Ile Pro Val Glu
235 240 245

AGG TAC TAC CGG ACT TAC CCG GCC AAA CCC CCA ACC GCA AGT TCT TTC 1120
Arg Tyr Tyr Arg Thr Tyr Pro Ala Lys Pro Pro Thr Ala Ser Ser Phe
250 255 260

GTC CTG CGC TCA GAG CCT GAA GCT GGA CAA GAA GAG GCA GAG TCA TCT 1168
Val Leu Arg Ser Glu Pro Glu Ala Gly Gln Glu Glu Ala Glu Ser Ser
265 270 275

GAG GCT GCT GCT GCT ACA CAA AAA GGG AGC CAG TCT GGA GAT ATC GGA 1216
Glu Ala Ala Ala Ala Thr Gln Lys Gly Ser Gln Ser Gly Asp Ile Gly
280 285 290

CTC ACT ACT GTG AGA ACC ATG AGA ACA TAT CAA GTT GAG GAC AAA AGT 1264
Leu Thr Thr Val Arg Thr Met Arg Thr Tyr Gln Val Glu Asp Lys Ser
295 300 305 310

GCA CCG GGT GGG AAA ATC GAC ATC GAA CGA GAT GAC CTC GCC AAA GGA 1312
Ala Pro Gly Gly Lys Ile Asp Ile Glu Arg Asp Asp Leu Ala Lys Gly
315 320 325

TAT GAG TAT GGA CGG ACA GCA GTT CAC ATT AGT GAA ACC GAC GAG AAC 1360
Tyr Glu Tyr Gly Arg Thr Ala Val His Ile Ser Glu Thr Asp Glu Asn
330 335 340

ATC ACG ATT CTC GAT ACA TTC GCA GGG CTG GAG TTG ATG GGC TTC ATC 1408
Ile Thr Ile Leu Asp Thr Phe Ala Gly Leu Glu Leu Met Gly Phe Ile
345 350 355

CAG ACT GAC CGG GTATGTCTTG CTGAAGTCGC CTCGGTGCAT GCTCTGACAC GATTAAT 1467
Gln Thr Asp Arg
360

TATAG TAT CAA CGT TAT ATG CAC ATG TCC AAC ACA AAC ATC ATA ATT GCA 1517
Tyr Gln Arg Tyr Met His Met Ser Asn Thr Asn Ile Ile Ile Ala
365 370 375

CAA CGT GCC AAC GAC AAA GCA GCC CTT GCC CTT TCA TCC TTT ATA CAC 1565
Gln Arg Ala Asn Asp Lys Ala Ala Leu Ala Leu Ser Ser Phe Ile His
380 385 390

GCG CTT TTT GAG CTA GAA TGC TAT GCT GTT GCT CGC CTA GTC GTG AAA 1613
Ala Leu Phe Glu Leu Glu Cys Tyr Ala Val Ala Arg Leu Val Val Lys
395 400 405

GAG AAC AAG CCA CCT GTT ATA GTC TTG CTC GCG CCC TCG ATC GAG CCT 1661
Glu Asn Lys Pro Pro Val Ile Val Leu Leu Ala Pro Ser Ile Glu Pro
410 415 420 425

GAT TAT GAA TGC CTT CTC GAA GTC CAG TTA CCA TTC GCG GAA GAT GTC 1709
Asp Tyr Glu Cys Leu Leu Glu Val Gln Leu Pro Phe Ala Glu Asp Val
430 435 440

CGA ACC TAT CGG TTC CCT CCT CTG GAT AAA GTG ATT ACT GTT TCT GGA 1757
Arg Thr Tyr Arg Phe Pro Pro Leu Asp Lys Val Ile Thr Val Ser Gly
445 450 455

AAG GTT GTG ACG CAA CAC CGG AAT CTT CCC AAT GAT GAT TTA CTC GAT 1805
Lys Val Val Thr Gln His Arg Asn Leu Pro Asn Asp Asp Leu Leu Asp
460 465 470

GTG ATG GGC AAG TAC GTG AAT AGT ATG GAG CTT GTC GAC GCA GAT GAG 1853
Val Met Gly Lys Tyr Val Asn Ser Met Glu Leu Val Asp Ala Asp Glu
475 480 485

GAT GG GTAGGTTTAT GCCTAAAAGA TTCCGAATCT CTTCTCATTG ACAAAACCAG G 1909
Asp Gl y
490

GAT CCA GTT GAG ACT TTC CCT ATC GAC GAC TCG TAT TCG CCT GTT TTG 1957
Asp Pro Val Glu Thr Phe Pro Ile Asp Asp Ser Tyr Ser Pro Val Leu
495 500 505

CAC CGG ATT GAC GCC GCC ATC CGT GCT CGG GCT ATA CAC CCT GAC CAG 2005
His Arg Ile Asp Ala Ala Ile Arg Ala Arg Ala Ile His Pro Asp Gln
510 515 520

CCC ATA CCT CCT CCA TCA GAG AGA CTG ACA AAA TTC TCA CAC CCA CGA 2053
Pro Ile Pro Pro Pro Ser Glu Arg Leu Thr Lys Phe Ser His Pro Arg
525 530 535

GAG GAT CTC ATC GAG AGA TCA CAG AAA TAC CTA GAG AAG TTG ATC GAG 2101
Glu Asp Leu Ile Glu Arg Ser Gln Lys Tyr Leu Glu Lys Leu Ile Glu
540 545 550 555

ATA GCC GAT GTT AAG AAG G GTTGGACATC ATCCCACAAT CAAGTCTATC AGGCTG 2156
Ile Ala Asp Val Lys Lys V
560

CTAATTCTGT TACCAG TT CCT CCC AAA GCG AAG GGT CGT AAG CGC ACT CGC 2207
al Pro Pro Lys Ala Lys Gly Arg Lys Arg Thr Arg
565 570

GAA ACT GAG AAG CCG CTT TCC GGA CTC GAC GTC GAT GCC CTG CTT CAT 2255
Glu Thr Glu Lys Pro Leu Ser Gly Leu Asp Val Asp Ala Leu Leu His
575 580 585

CAT GAA AAG CGC GCC AAG ATA TCT CCC AAC AAT GCC ATT CCC GAG TTC 2303
His Glu Lys Arg Ala Lys Ile Ser Pro Asn Asn Ala Ile Pro Glu Phe
590 595 600 605

AAG CAG ACT CTC GCA CAG GCC GAG AAT ATC GAG GCC ATC AAA GAC GCT 2351
Lys Gln Thr Leu Ala Gln Ala Glu Asn Ile Glu Ala Ile Lys Asp Ala
610 615 620

ACA AAG CAG ATG ATG GTT ATC GTT GAA GAT CAA ATC AAA CAC AGT CTC 2399
Thr Lys Gln Met Met Val Ile Val Glu Asp Gln Ile Lys His Ser Leu
625 630 635

GGT GAT GCT AAC TAC GAC CGG GTC ATT GAA GCG CTG GGC ACG ATG CGT 2447
Gly Asp Ala Asn Tyr Asp Arg Val Ile Glu Ala Leu Gly Thr Met Arg
640 645 650

GAC GAG TTG GTA TCA TAC GAA GAG CCT ACC TCC TAC AAT AAC TTC CTA 2495
Asp Glu Leu Val Ser Tyr Glu Glu Pro Thr Ser Tyr Asn Asn Phe Leu
655 660 665

GGC CAG CTC AAG GAT AAG TTG TTA CAG GAG AAG CTT GGA GGA GAT CGA 2543
Gly Gln Leu Lys Asp Lys Leu Leu Gln Glu Lys Leu Gly Gly Asp Arg
670 675 680 685

CAA GAG CTG TGG TGG CTT ATT CGA CGA AAC AAG CTG GGA CTT GTC ACT 2591
Gln Glu Leu Trp Trp Leu Ile Arg Arg Asn Lys Leu Gly Leu Val Thr
690 695 700

CAG CGC GAG TCG GAT CAA TCT AGG GTT ACC GAT ACG GAA GCC AAA GAA 2639
Gln Arg Glu Ser Asp Gln Ser Arg Val Thr Asp Thr Glu Ala Lys Glu
705 710 715

GTA AGT CTC ATT AAG ATG AAG GAG TGA 2666
Val Ser Leu Ile Lys Met Lys Glu Stop
720

<210> 4
<211> 2249
<212> DNA
<213> Aspergillus oryzae

<220>
<223> Aspergillus oryzae Ku70

<400> 4
ATG GCT GAC GAG GAT CAA TAT CGT GGA GAC GAC CAG ATC GAT GAG GAA 48
Met Ala Asp Glu Asp Gln Tyr Arg Gly Asp Asp Gln Ile Asp Glu Glu
1               5                   10                  15

GAG GAG GAG ACC GAC GAG AGT GTACACACTT TCAAACACAC CTGAAAGCTT CGGA 103
Glu Glu Glu Thr Asp Glu Ser
            20

GGCTAACATG TTATCAACCA AAATAG GGA TAC AAA ACA GTG AAA GAT GCC GTT 156
Gly Tyr Lys Thr Val Lys Asp Ala Val
    25                  30

CTT TTT GCT ATC GAA GTC AGC GAT TCG ATG CTC ACG CCT CGT CCA TCT 204
Leu Phe Ala Ile Glu Val Ser Asp Ser Met Leu Thr Pro Arg Pro Ser
        35                  40                  45

TCC GAT TCA AAG AAA CCT GCG GAG GAG TCC CCC ACA ACG GCC GCA CTA 252
Ser Asp Ser Lys Lys Pro Ala Glu Glu Ser Pro Thr Thr Ala Ala Leu
    50                  55                  60

AAA TGC GCA TAT TAT CTC ATG CAA CAA CGC ATT ATC TCT AAT CCC CGT 300
Lys Cys Ala Tyr Tyr Leu Met Gln Gln Arg Ile Ile Ser Asn Pro Arg
65                  70                  75                  80

GAC ATG ATC GGT GTG CTA TTA TAT GGG ACG CAG GCG TCC AAA TTT TAT 348
Asp Met Ile Gly Val Leu Leu Tyr Gly Thr Gln Ala Ser Lys Phe Tyr
                85                  90                  95

GAC GAG GAT GAA AAT AGT CGA GGA GAT CTT TCA TAC CCA CAC TGC TAC 396
Asp Glu Asp Glu Asn Ser Arg Gly Asp Leu Ser Tyr Pro His Cys Tyr
            100                 105                 110

CTT TTC ACA GAC CTT GAT GTC CCC TCT GCG CAA GAA GTC AAG AAT CTT 444
Leu Phe Thr Asp Leu Asp Val Pro Ser Ala Gln Glu Val Lys Asn Leu
        115                 120                 125

CGG GCA CTA GCA CAA GAC GGC GAT GAA TCA AAG GAT GTA CTT AAG GCG 492
Arg Ala Leu Ala Gln Asp Gly Asp Glu Ser Lys Asp Val Leu Lys Ala
    130                 135                 140

TCA GGC GAG CGG GTC TCA ATG GCG AAC GTA CTC TTT TGC GCC AAT CAA 540
Ser Gly Glu Arg Val Ser Met Ala Asn Val Leu Phe Cys Ala Asn Gln
145                 150                 155                 160

ATA TTC ACG TCG AAA GCC CCT AAC TTC TTG TCT CGG CGA TTG TTT ATA 588
Ile Phe Thr Ser Lys Ala Pro Asn Phe Leu Ser Arg Arg Leu Phe Ile
                165                 170                 175

GTC ACC GAT AAT GAT GAC CCT CAT GGC GAT AAT AAA AGC TTG AGA TCC 636
Val Thr Asp Asn Asp Asp Pro His Gly Asp Asn Lys Ser Leu Arg Ser
            180                 185                 190

GCT TCA ACT GTA CGC GCG AAG GAC TTA TAT GAC CTC GGT GTC ACT ATT 684
Ala Ser Thr Val Arg Ala Lys Asp Leu Tyr Asp Leu Gly Val Thr Ile
        195                 200                 205

GAG CTG TTT CCG ATT TCT CGG CCA GGC CAT GAG TTC GAT ACC GCC AGA 732
Glu Leu Phe Pro Ile Ser Arg Pro Gly His Glu Phe Asp Thr Ala Arg
    210                 215                 220

TTC TAT GAC GTAAGATTA TATTGACTCA ATGTGAAGTA TCGCTGCTAA CAGCAATTAG 790
Phe Tyr Asp
225

GAT ATC ATC TAC AAG GCC TCT CCT TCG GAT CCA GAT GCC CCG GCA TAC 838
Asp Ile Ile Tyr Lys Ala Ser Pro Ser Asp Pro Asp Ala Pro Ala Tyr
        230                 235                 240

CTG CAA ACC GAT TCC AAG GCT TCT CCA GCC ACC GGG GAT GGG ATA TCA 886
Leu Gln Thr Asp Ser Lys Ala Ser Pro Ala Thr Gly Asp Gly Ile Ser
    245                 250                 255

CTG CTC AAT ACC CTC CTG TCC AAT ATC AAT TCA AGA TCT GTC CCA CGG 934
Leu Leu Asn Thr Leu Leu Ser Asn Ile Asn Ser Arg Ser Val Pro Arg
260                 265                 270                 275

CGT GCA CAG TTC TCC AAT ATA CCA TTG GAG CTT GGA CCA AAC TTA AAA 982
Arg Ala Gln Phe Ser Asn Ile Pro Leu Glu Leu Gly Pro Asn Leu Lys
                280                 285                 290

ATA TCT GTC TCA GGA TAT CTT TTG TTC AAG CGT CAA GCA CCC GCC AGA 1030
Ile Ser Val Ser Gly Tyr Leu Leu Phe Lys Arg Gln Ala Pro Ala Arg
            295                 300                 305

AAC TCC TTC ATC TGG CTC GGC GGT GAA CAG CCC CAG ATT GTC AAA GGA 1078
Asn Ser Phe Ile Trp Leu Gly Gly Glu Gln Pro Gln Ile Val Lys Gly
        310                 315                 320

GTG ACC ACT CAA ATC GCT GAC GAC ACG GCT CGC ACG ATT GAA AAG TGG 1126
Val Thr Thr Gln Ile Ala Asp Asp Thr Ala Arg Thr Ile Glu Lys Trp
    325                 330                 335

GAA ATT AAG AAA GCT TAT AAG TTT GGC GGT GAT CAG GTT GCT TTC ACG 1174
Glu Ile Lys Lys Ala Tyr Lys Phe Gly Gly Asp Gln Val Ala Phe Thr
340                 345                 350                 355

CCC GAA GAG ATG AAG TCA CTG AGG AAC TTC GGT GAT CCT GTC ATC CGT 1222
Pro Glu Glu Met Lys Ser Leu Arg Asn Phe Gly Asp Pro Val Ile Arg
                360                 365                 370

ATA ATA GGG TTC AAG CCC CTC TCT GCA CTT CCG TTC TGG GCC AAT ATC 1270
Ile Ile Gly Phe Lys Pro Leu Ser Ala Leu Pro Phe Trp Ala Asn Ile
            375                 380                 385

AAA CAC CCC TCC TTT ATA TAC CCA TCG GAA GAA GAT TTT GTG GGC TCC 1318
Lys His Pro Ser Phe Ile Tyr Pro Ser Glu Glu Asp Phe Val Gly Ser
        390                 395                 400

ACG CGG GTT TTT TCT GCT TTG CAT CAG ACA CTC CTC CGG GAT AAA AAG 1366
Thr Arg Val Phe Ser Ala Leu His Gln Thr Leu Leu Arg Asp Lys Lys
    405                 410                 415

GCC GCA CTT GTC TGG TTC ATT GCT CGT AAA AAT GCA AGT CCT GTT CTG 1414
Ala Ala Leu Val Trp Phe Ile Ala Arg Lys Asn Ala Ser Pro Val Leu
420                 425                 430                 435

GGG GCT ATG GTC GCC GGA GAA GAG AAA CTA GAC GAG AGT GGC GTC CAG 1462
Gly Ala Met Val Ala Gly Glu Glu Lys Leu Asp Glu Ser Gly Val Gln
                440                 445                 450

AAG TTT CCT CCA GGA ATG TGG ATA ATA CCT CTC CCG TTC GCT GAT GAC 1510
Lys Phe Pro Pro Gly Met Trp Ile Ile Pro Leu Pro Phe Ala Asp Asp
            455                 460                 465

GTC CGT CAA AAC CCT GAA ACC ACA CTC CAT GTT GCA CCT GAG CCA TTG 1558
Val Arg Gln Asn Pro Glu Thr Thr Leu His Val Ala Pro Glu Pro Leu
        470                 475                 480

ATC GAT CAA ATG CGG TAT ATT GTC CAG CAA TTG CAA CTT CCA AAG GCG 1606
Ile Asp Gln Met Arg Tyr Ile Val Gln Gln Leu Gln Leu Pro Lys Ala
    485                 490                 495

TCT TAC GAC CCC TTT AAG TAC CCT AAT CCA T GTAAGCTTCT GCCAACTTCC 1657
Ser Tyr Asp Pro Phe Lys Tyr Pro Asn Pro S
500 505 510

TGCACAGAAA CTCTGGCATT AACCTATTGC TCTGTTAG CC CTC CAA TGG CAT 1709
er Leu Gln Trp His

TAT CGC ATT CTA CAA GCC TTG GCG TTG GAT GAG GAC CTC CCG GAG AAG 1757
Tyr Arg Ile Leu Gln Ala Leu Ala Leu Asp Glu Asp Leu Pro Glu Lys
515 520 525 530

CCA GAA GAC AAA ACG TTG CCC AGA TAT CGG CAG ATC GAT AAA GTATATC 1806
Pro Glu Asp Lys Thr Leu Pro Arg Tyr Arg Gln Ile Asp Lys
535 540

ACACATTCCT ATTCTTTCCA CGGATCTTGC TGACCTTCGC TTAG CGC ACT GGC 1859
Arg Thr Gly
545

GAC TAT GTA TTG TCT TGG GCC GAC GAG TTG GAA AAG CAA TAC GCG AAA 1907
Asp Tyr Val Leu Ser Trp Ala Asp Glu Leu Glu Lys Gln Tyr Ala Lys
550 555 560

ATA TCG GCA CAT GGC CCG AAG AGC ACA CTC GTC AAA CGA AGC GCC AAA 1955
Ile Ser Ala His Gly Pro Lys Ser Thr Leu Val Lys Arg Ser Ala Lys
565 570 575

GAC CGA ACA TCT GAA GTC GAG GAT GCA GCC CAG AAG CCA TAC AAG AAA 2003
Asp Arg Thr Ser Glu Val Glu Asp Ala Ala Gln Lys Pro Tyr Lys Lys
580 585 590 595

GTG AAG GTG GAG ACA GAC GAG CAA GGC GTT GAA GAT GTA GTG CGA GCC     2051
Val Lys Val Glu Thr Asp Glu Gln Gly Val Glu Asp Val Val Arg Ala
                600                 605                 610

CAT TAC CAG AAG GGA TCG CTA TCG AAG GTGACTATTA CCTGCCCCCT AGGCT     2103
His Tyr Gln Lys Gly Ser Leu Ser Lys
            615                 620

TTAATTTGGA CTAACTAACG  CGCGTGACTT GTGTGTAG  CTT ACG GTA CCT GTC     2156
                                            Leu Thr Val Pro Val
                                                            625

CTC AAA GAC TTT CTG AAT GCC CAT GGA CGC TCC GCT GCT GGG AAG AAA     2204
Leu Lys Asp Phe Leu Asn Ala His Gly Arg Ser Ala Ala Gly Lys Lys
                630                 635                 640

GCT GAT CTC GTT GAG CGT GTG GAG GAG TAT TTG GAG CAG AAA TGA         2249
Ala Asp Leu Val Glu Arg Val Glu Glu Tyr Leu Glu Gln Lys Sto
            645                 650                 655

<210> 5
<211> 2703
<212> DNA
<213> Aspergillus oryzae

<220>
<223> Aspergillus oryzae Ku80 Long

<400> 5
ATG GCG GAC AAG GAA GCA ACT GTG TAT ATT GTG GAC GTT GGG AGG TCC       48
Met Ala Asp Lys Glu Ala Thr Val Tyr Ile Val Asp Val Gly Arg Ser
  1               5                  10                  15

ATG GGA GAA TGT CGC AAT GGC CGA TCA GTG ACT GAT CTT GAA TGG GCC       96
Met Gly Glu Cys Arg Asn Gly Arg Ser Val Thr Asp Leu Glu Trp Ala
             20                  25                  30

ATG CAG TAT GTC TGG GAT CGC ATT ACA GGA ACA GTGAGTGGCA GTCGTCACAA    149
Met Gln Tyr Val Trp Asp Arg Ile Thr Gly Thr
         35                  40

TTGGACCGTA TTCGTTAAAT ACCTTGCTCA ATTTCAAACC AG GTG GCC ACT GGC       203
                                               Val Ala Thr Gly
                                                        45

CGC AAA ACT GCC ATG ATG GGT GTG ATT GGA CTC AGG ACA GAT G GTATGTA    253
Arg Lys Thr Ala Met Met Gly Val Ile Gly Leu Arg Thr Asp G
         50                  55                  60

TACTTCTGAA TACTGTATGC GGTTCATACG CTGATCCAAA AATTAG AA ACG TCC AAC    310
                                                   lu Thr Ser Asn
                                                               65

GAA CTT GAA GAT GAC GTA CAT TTC TCT CAC ATT GCA GTT CTG TCG AAC      358
Glu Leu Glu Asp Asp Val His Phe Ser His Ile Ala Val Leu Ser Asn
                 70                  75                  80

CTC AAA CA GTATGCTTTC CACTCTATGA TAATTTGGTT TGTGCGCCAA ACTGACGAGG    416
Leu Lys Gl

ACGTCAAG G TTT CTT ATG CCG GAC ATT CGG AAA CTG GAA GAT GAA CTG       464
         n Phe Leu Met Pro Asp Ile Arg Lys Leu Glu Asp Glu Leu
             85                  90                  95

AAA CCG AGC AAA ACG GAC AAA GGA GAC G GTAAGCTTTT TGAGAGCCAC TAGGA    517
Lys Pro Ser Lys Thr Asp Lys Gly Asp A
        100                 105

CCTACTGTCC AATTTACTAA ACTTTGTTCT CTAG CT ATT TCC GCT ATT ATC TTG     571
                                      la Ile Ser Ala Ile Ile Leu
                                                         110

GCT ATT CAG ATG ATT ATC ACG CAT TGC AAG AAG TTG AAG TAC AGG CGC      619
Ala Ile Gln Met Ile Ile Thr His Cys Lys Lys Leu Lys Tyr Arg Arg
    115                 120                 125

AAG ATC GTC CTC GTC ACT AAC GGA CAG GGG CGC ATG AGC GAT GAA GAC 667
Lys Ile Val Leu Val Thr Asn Gly Gln Gly Arg Met Ser Asp Glu Asp
130 135 140 145

CTG GGC GAG ATT GTG AAG AAG GTC AAG GAA GAT AAC ATC GAG CTT GTT 715
Leu Gly Glu Ile Val Lys Lys Val Lys Glu Asp Asn Ile Glu Leu Val
150 155 160

GTT AT GTCAGTGATT TGCTACCAGA TAGCAACGAA ACAAAAGCTA ACTTCAAGCA G G 772
Val Me t

GGA ATT GAT TTC GAT GAC CCT GAG TAC GGT TAC AAA GAA GAA GAC AAA 820
Gly Ile Asp Phe Asp Asp Pro Glu Tyr Gly Tyr Lys Glu Glu Asp Lys
165 170 175

GAC CCT CAC AAG GTAGCGATAT CTCTCGCGCA GCTTTGTTCT TTTCTAACAA CTAAA 877
Asp Pro His Lys
180

ACAG GCC GAA AAT GAA ACT CTC TTG CGT ACC CTT GTG GAA GAT TGT GAT 926
Ala Glu Asn Glu Thr Leu Leu Arg Thr Leu Val Glu Asp Cys Asp
185 190 195

GGA GTC TAT GGA ACA TTC GAG CAG GCT GTG GCT GAA CTA GAC ATC CCC 974
Gly Val Tyr Gly Thr Phe Glu Gln Ala Val Ala Glu Leu Asp Ile Pro
200 205 210

CGT GTC AAG TCT GTC AGG TCA GTG GCA AGC TTC AAA GGA TAT CTC CAA 1022
Arg Val Lys Ser Val Arg Ser Val Ala Ser Phe Lys Gly Tyr Leu Gln
215 220 225 230

CTA GGC AAC CCA GAG GAG TAT GAC TCT GCT CTC CGC ATT CCT GTT GAA 1070
Leu Gly Asn Pro Glu Glu Tyr Asp Ser Ala Leu Arg Ile Pro Val Glu
235 240 245

AGG TAC TAT CGG ACT TAC CCG GCC AAA CCC CCG ACC GCA AGT TCT TTC 1118
Arg Tyr Tyr Arg Thr Tyr Pro Ala Lys Pro Pro Thr Ala Ser Ser Phe
250 255 260

GTC CTG CGC TCA GAG CCT GAA GCT GGA CAA GAA GAG GCA GAG TCA TCT 1166
Val Leu Arg Ser Glu Pro Glu Ala Gly Gln Glu Glu Ala Glu Ser Ser
265 270 275

GAG GCT GCT GCT GCT ACG CAA AAA GGG AGC CAA TCT GGA GAT GCC GGA 1214
Glu Ala Ala Ala Ala Thr Gln Lys Gly Ser Gln Ser Gly Asp Ala Gly
280 285 290

TTG ACC ACT GTG AGA ACC ATG AGA ACA TAT CAA GTT GAG GAC AAA AGT 1262
Leu Thr Thr Val Arg Thr Met Arg Thr Tyr Gln Val Glu Asp Lys Ser
295 300 305 310

GCA CCG GGT GGG AAA ATC GAC ATC GAA CGA GAT GAG CTC GCC AAA GGA 1310
Ala Pro Gly Gly Lys Ile Asp Ile Glu Arg Asp Glu Leu Ala Lys Gly
315 320 325

TAT GAG TAT GGA CGG ACA GCA GTT CAC ATT AGT GAA ACT GAC GAG AAC 1358
Tyr Glu Tyr Gly Arg Thr Ala Val His Ile Ser Glu Thr Asp Glu Asn
330 335 340

ATC ACG ATT CTC GAT ACA TTC GCA GGG CTG GAG TTG ATG GGC TTC ATC 1406
Ile Thr Ile Leu Asp Thr Phe Ala Gly Leu Glu Leu Met Gly Phe Ile
345 350 355

CAG ACT GAC CAG GTATGTCTTG CTGAAGTCGC CTCGGTGCAT GCTCTGACAC ACGAT 1463
Gln Thr Asp Gln
360

TATAG TAT CAA CGT TAT ATG CAC ATG TCC AAC ACA AAC ATC ATA ATT GCA 1513
Tyr Gln Arg Tyr Met His Met Ser Asn Thr Asn Ile Ile Ile Ala
365 370 375

CAA CGT GCC AAC GAC AAA GCA GCT CTT GCC CTT TCA TCC TTT ATA CAC 1561
Gln Arg Ala Asn Asp Lys Ala Ala Leu Ala Leu Ser Ser Phe Ile His
380 385 390

GCC CTT TTT GAG CTA GAA TGC TAT GCT GTT GCT CGC CTA GTT GTG AAA 1609
Ala Leu Phe Glu Leu Glu Cys Tyr Ala Val Ala Arg Leu Val Val Lys
395 400 405

GAG AAC AAG CCA CCA GTT ATA GTC TTG CTC GCG CCC TCG ATC GAG CCT 1657
Glu Asn Lys Pro Pro Val Ile Val Leu Leu Ala Pro Ser Ile Glu Pro
410 415 420 425

GAG TAT GAA TGC CTT CTC GAA GTC CAG TTA CCA TTT GCG GAA GAT GTC 1705
Glu Tyr Glu Cys Leu Leu Glu Val Gln Leu Pro Phe Ala Glu Asp Val
430 435 440

CGA ACC TAT CGG TTC CCT CCT CTG GAT AAA GTG ATT ACT GTT TCC GGA 1753
Arg Thr Tyr Arg Phe Pro Pro Leu Asp Lys Val Ile Thr Val Ser Gly
445 450 455

AAG GTT GTG ACA CAA CAC CGG AAT CTT CCC AGT GAT GAT TTA CTC GAT 1801
Lys Val Val Thr Gln His Arg Asn Leu Pro Ser Asp Asp Leu Leu Asp
460 465 470

GTG ATG GGC AAG TAC GTG AAT AGT ATG GAG CTT GTC GAC GCA GAT GAG 1849
Val Met Gly Lys Tyr Val Asn Ser Met Glu Leu Val Asp Ala Asp Glu
475 480 485

GAT GG GTAGGTTTAT GCCTAAAAGA TTCCGAATAT CTTCTCATTG ACATAACCAG G 1905
Asp Gl y
490

GAT CCA GTG GAG ACT TTC CCT ATC GAC GAC TCG TAT TCC CCA GTT TTG 1953
Asp Pro Val Glu Thr Phe Pro Ile Asp Asp Ser Tyr Ser Pro Val Leu
495 500 505

CAC CGG ATT GAC GCC GCC ATC CGT GCT CGG GCT ATA CAT CCT GAC CAG 2001
His Arg Ile Asp Ala Ala Ile Arg Ala Arg Ala Ile His Pro Asp Gln
510 515 520

CCC ATA CCT CCT CCA TCA GAG AGA CTG ACA AAA TTC TCA CAC CCA CGA 2049
Pro Ile Pro Pro Pro Ser Glu Arg Leu Thr Lys Phe Ser His Pro Arg
525 530 535

GAG GAT CTC ATC GAG AAA TCA CAG AAA CAC CTA GAG AAG TTG ATC GAG 2097
Glu Asp Leu Ile Glu Lys Ser Gln Lys His Leu Glu Lys Leu Ile Glu
540 545 550 555

ATA GCC GAT GTT AAG AAG G GTTGGACATC ACCCCACAAT CAAGTCTGTC AGACTG 2152
Ile Ala Asp Val Lys Lys V
560

CTAATTCAGT TACCAG TT CCT CCC AAA GCG AAG GGT CGC AAG CGC ACT CGT 2203
al Pro Pro Lys Ala Lys Gly Arg Lys Arg Thr Arg
565 570

GAA ACC GAA AAG CCA CTT TCC GGA CTC GAC GTC GAT GCC CTG CTT CAT 2251
Glu Thr Glu Lys Pro Leu Ser Gly Leu Asp Val Asp Ala Leu Leu His
575 580 585

CAT GAA AAG CGC GTC AAG ATA TCT CCC AAC AAT GCC ATT CCT GAG TTC 2299
His Glu Lys Arg Val Lys Ile Ser Pro Asn Asn Ala Ile Pro Glu Phe
590 595 600 605

AAG CAG ACT CTC GCA CAG GCC GAG AAT ATC GAG GCC ATC AAA GAC GCT 2347
Lys Gln Thr Leu Ala Gln Ala Glu Asn Ile Glu Ala Ile Lys Asp Ala
610 615 620

ACA AAG CAG ATG ATG GTC ATC GTT GAA GAT CAA ATC AAA CAC AGT CTC 2395
Thr Lys Gln Met Met Val Ile Val Glu Asp Gln Ile Lys His Ser Leu
625 630 635

GGT AAT GCT AAC TAC GAC CGG GTC ATT GAA GCG CTG GGC ACG ATG CGT 2443
Gly Asn Ala Asn Tyr Asp Arg Val Ile Glu Ala Leu Gly Thr Met Arg
640 645 650

GAC GAG TTG GTA TCT TAC GAA GAG CCT GCC TCC TAC AAT GAC TTC CTG 2491
Asp Glu Leu Val Ser Tyr Glu Glu Pro Ala Ser Tyr Asn Asp Phe Leu
655 660 665

GGC CAG CTC AAG GAT AAG TTA CTG CAG GAG AAG CTT GGA GGA GAC CGA 2539
Gly Gln Leu Lys Asp Lys Leu Leu Gln Glu Lys Leu Gly Gly Asp Arg
670 675 680 685

CAA GAG CTG TGG TGG CTT GTT CGA CGA AAC AAG CTG GGA CTT GTC ACT 2587
Gln Glu Leu Trp Trp Leu Val Arg Arg Asn Lys Leu Gly Leu Val Thr
690 695 700

CAG CGC GAG TCG GAT CAA TCT AGG GTT ACC GAT ACG GAA GCC AAA GAA G 2636
Gln Arg Glu Ser Asp Gln Ser Arg Val Thr Asp Thr Glu Ala Lys Glu
705 710 715

TAAGTCTCAC TAAGATGAAG GAGTGACCTA GACTAACTGC AGTTTACAG TTC ATG TCC 2694
Phe Met Ser
720

GCC AGA TGA 2703
Ala Arg Stop

<210> 6
<211> 2662
<212> DNA
<213> Aspergillus oryzae

<220>
<223> Aspergillus oryzae Ku80 Short

<400> 6
ATG GCG GAC AAG GAA GCA ACT GTG TAT ATT GTG GAC GTT GGG AGG TCC 48
Met Ala Asp Lys Glu Ala Thr Val Tyr Ile Val Asp Val Gly Arg Ser
1               5                   10                  15

ATG GGA GAA TGT CGC AAT GGC CGA TCA GTG ACT GAT CTT GAA TGG GCC 96
Met Gly Glu Cys Arg Asn Gly Arg Ser Val Thr Asp Leu Glu Trp Ala
            20                  25                  30

ATG CAG TAT GTC TGG GAT CGC ATT ACA GGA ACA GTGAGTGGCA GTCGTCACAA 149
Met Gln Tyr Val Trp Asp Arg Ile Thr Gly Thr
        35                  40

TTGGACCGTA TTCGTTAAAT ACCTTGCTCA ATTTCAAACC AG GTG GCC ACT GGC 203
Val Ala Thr Gly
    45

CGC AAA ACT GCC ATG ATG GGT GTG ATT GGA CTC AGG ACA GAT G GTATGTA 253
Arg Lys Thr Ala Met Met Gly Val Ile Gly Leu Arg Thr Asp G
50 55 60

TACTTCTGAA TACTGTATGC GGTTCATACG CTGATCCAAA AATTAG AA ACG TCC AAC 310
lu Thr Ser Asn
65

GAA CTT GAA GAT GAC GTA CAT TTC TCT CAC ATT GCA GTT CTG TCG AAC 358
Glu Leu Glu Asp Asp Val His Phe Ser His Ile Ala Val Leu Ser Asn
70 75 80

CTC AAA CA GTATGCTTTC CACTCTATGA TAATTTGGTT TGTGCGCCAA ACTGACGAGG 416
Leu Lys Gl

ACGTCAAG G TTT CTT ATG CCG GAC ATT CGG AAA CTG GAA GAT GAA CTG 464
n Phe Leu Met Pro Asp Ile Arg Lys Leu Glu Asp Glu Leu
85 90 95

AAA CCG AGC AAA ACG GAC AAA GGA GAC G GTAAGCTTTT TGAGAGCCAC TAGGA 517
Lys Pro Ser Lys Thr Asp Lys Gly Asp A
100 105

CCTACTGTCC AATTTACTAA ACTTTGTTCT CTAG CT ATT TCC GCT ATT ATC TTG 571
la Ile Ser Ala Ile Ile Leu
110

GCT ATT CAG ATG ATT ATC ACG CAT TGC AAG AAG TTG AAG TAC AGG CGC 619
Ala Ile Gln Met Ile Ile Thr His Cys Lys Lys Leu Lys Tyr Arg Arg

115                 120                 125

AAG ATC GTC CTC GTC ACT AAC GGA CAG GGG CGC ATG AGC GAT GAA GAC          667
Lys Ile Val Leu Val Thr Asn Gly Gln Gly Arg Met Ser Asp Glu Asp
130                 135                 140                 145

CTG GGC GAG ATT GTG AAG AAG GTC AAG GAA GAT AAC ATC GAG CTT GTT          715
Leu Gly Glu Ile Val Lys Lys Val Lys Glu Asp Asn Ile Glu Leu Val
                150                 155                 160

GTT AT GTCAGTGATT TGCTACCAGA TAGCAACGAA ACAAAAGCTA ACTTCAAGCA G G        772
Val Me                                                          t

GGA ATT GAT TTC GAT GAC CCT GAG TAC GGT TAC AAA GAA GAA GAC AAA          820
Gly Ile Asp Phe Asp Asp Pro Glu Tyr Gly Tyr Lys Glu Glu Asp Lys
    165                 170                 175

GAC CCT CAC AAG GTAGCGATAT CTCTCGCGCA GCTTTGTTCT TTTCTAACAA CTAAA        877
Asp Pro His Lys
180

ACAG GCC GAA AAT GAA ACT CTC TTG CGT ACC CTT GTG GAA GAT TGT GAT         926
     Ala Glu Asn Glu Thr Leu Leu Arg Thr Leu Val Glu Asp Cys Asp
         185                 190                 195

GGA GTC TAT GGA ACA TTC GAG CAG GCT GTG GCT GAA CTA GAC ATC CCC          974
Gly Val Tyr Gly Thr Phe Glu Gln Ala Val Ala Glu Leu Asp Ile Pro
    200                 205                 210

CGT GTC AAG TCT GTC AGG TCA GTG GCA AGC TTC AAA GGA TAT CTC CAA          1022
Arg Val Lys Ser Val Arg Ser Val Ala Ser Phe Lys Gly Tyr Leu Gln
215                 220                 225                 230

CTA GGC AAC CCA GAG GAG TAT GAC TCT GCT CTC CGC ATT CCT GTT GAA          1070
Leu Gly Asn Pro Glu Glu Tyr Asp Ser Ala Leu Arg Ile Pro Val Glu
                235                 240                 245

AGG TAC TAT CGG ACT TAC CCG GCC AAA CCC CCG ACC GCA AGT TCT TTC          1118
Arg Tyr Tyr Arg Thr Tyr Pro Ala Lys Pro Pro Thr Ala Ser Ser Phe
            250                 255                 260

GTC CTG CGC TCA GAG CCT GAA GCT GGA CAA GAA GAG GCA GAG TCA TCT          1166
Val Leu Arg Ser Glu Pro Glu Ala Gly Gln Glu Glu Ala Glu Ser Ser
        265                 270                 275

GAG GCT GCT GCT GCT ACG CAA AAA GGG AGC CAA TCT GGA GAT GCC GGA          1214
Glu Ala Ala Ala Ala Thr Gln Lys Gly Ser Gln Ser Gly Asp Ala Gly
    280                 285                 290

TTG ACC ACT GTG AGA ACC ATG AGA ACA TAT CAA GTT GAG GAC AAA AGT          1262
Leu Thr Thr Val Arg Thr Met Arg Thr Tyr Gln Val Glu Asp Lys Ser
295                 300                 305                 310

GCA CCG GGT GGG AAA ATC GAC ATC GAA CGA GAT GAG CTC GCC AAA GGA          1310
Ala Pro Gly Gly Lys Ile Asp Ile Glu Arg Asp Glu Leu Ala Lys Gly
                315                 320                 325

TAT GAG TAT GGA CGG ACA GCA GTT CAC ATT AGT GAA ACT GAC GAG AAC          1358
Tyr Glu Tyr Gly Arg Thr Ala Val His Ile Ser Glu Thr Asp Glu Asn
            330                 335                 340

ATC ACG ATT CTC GAT ACA TTC GCA GGG CTG GAG TTG ATG GGC TTC ATC          1406
Ile Thr Ile Leu Asp Thr Phe Ala Gly Leu Glu Leu Met Gly Phe Ile
        345                 350                 355

CAG ACT GAC CAG GTATGTCTTG CTGAAGTCGC CTCGGTGCAT GCTCTGACAC ACGAT        1463
Gln Thr Asp Gln
    360

TATAG TAT CAA CGT TAT ATG CAC ATG TCC AAC ACA AAC ATC ATA ATT GCA        1513
      Tyr Gln Arg Tyr Met His Met Ser Asn Thr Asn Ile Ile Ile Ala
              365                 370                 375

CAA CGT GCC AAC GAC AAA GCA GCT CTT GCC CTT TCA TCC TTT ATA CAC          1561
Gln Arg Ala Asn Asp Lys Ala Ala Leu Ala Leu Ser Ser Phe Ile His

380 385 390

GCC CTT TTT GAG CTA GAA TGC TAT GCT GTT GCT CGC CTA GTT GTG AAA 1609
Ala Leu Phe Glu Leu Glu Cys Tyr Ala Val Ala Arg Leu Val Val Lys
395 400 405

GAG AAC AAG CCA CCA GTT ATA GTC TTG CTC GCG CCC TCG ATC GAG CCT 1657
Glu Asn Lys Pro Pro Val Ile Val Leu Leu Ala Pro Ser Ile Glu Pro
410 415 420 425

GAG TAT GAA TGC CTT CTC GAA GTC CAG TTA CCA TTT GCG GAA GAT GTC 1705
Glu Tyr Glu Cys Leu Leu Glu Val Gln Leu Pro Phe Ala Glu Asp Val
430 435 440

CGA ACC TAT CGG TTC CCT CCT CTG GAT AAA GTG ATT ACT GTT TCC GGA 1753
Arg Thr Tyr Arg Phe Pro Pro Leu Asp Lys Val Ile Thr Val Ser Gly
445 450 455

AAG GTT GTG ACA CAA CAC CGG AAT CTT CCC AGT GAT GAT TTA CTC GAT 1801
Lys Val Val Thr Gln His Arg Asn Leu Pro Ser Asp Asp Leu Leu Asp
460 465 470

GTG ATG GGC AAG TAC GTG AAT AGT ATG GAG CTT GTC GAC GCA GAT GAG 1849
Val Met Gly Lys Tyr Val Asn Ser Met Glu Leu Val Asp Ala Asp Glu
475 480 485

GAT GG GTAGGTTTAT GCCTAAAAGA TTCCGAATAT CTTCTCATTG ACATAACCAG G 1905
Asp Gl y
490

GAT CCA GTG GAG ACT TTC CCT ATC GAC GAC TCG TAT TCC CCA GTT TTG 1953
Asp Pro Val Glu Thr Phe Pro Ile Asp Asp Ser Tyr Ser Pro Val Leu
495 500 505

CAC CGG ATT GAC GCC GCC ATC CGT GCT CGG GCT ATA CAT CCT GAC CAG 2001
His Arg Ile Asp Ala Ala Ile Arg Ala Arg Ala Ile His Pro Asp Gln
510 515 520

CCC ATA CCT CCT CCA TCA GAG AGA CTG ACA AAA TTC TCA CAC CCA CGA 2049
Pro Ile Pro Pro Pro Ser Glu Arg Leu Thr Lys Phe Ser His Pro Arg
525 530 535

GAG GAT CTC ATC GAG AAA TCA CAG AAA CAC CTA GAG AAG TTG ATC GAG 2097
Glu Asp Leu Ile Glu Lys Ser Gln Lys His Leu Glu Lys Leu Ile Glu
540 545 550 555

ATA GCC GAT GTT AAG AAG G GTTGGACATC ACCCCACAAT CAAGTCTGTC AGACTG 2152
Ile Ala Asp Val Lys Lys V
560

CTAATTCAGT TACCAG TT CCT CCC AAA GCG AAG GGT CGC AAG CGC ACT CGT 2203
al Pro Pro Lys Ala Lys Gly Arg Lys Arg Thr Arg
565 570

GAA ACC GAA AAG CCA CTT TCC GGA CTC GAC GTC GAT GCC CTG CTT CAT 2251
Glu Thr Glu Lys Pro Leu Ser Gly Leu Asp Val Asp Ala Leu Leu His
575 580 585

CAT GAA AAG CGC GTC AAG ATA TCT CCC AAC AAT GCC ATT CCT GAG TTC 2299
His Glu Lys Arg Val Lys Ile Ser Pro Asn Asn Ala Ile Pro Glu Phe
590 595 600 605

AAG CAG ACT CTC GCA CAG GCC GAG AAT ATC GAG GCC ATC AAA GAC GCT 2347
Lys Gln Thr Leu Ala Gln Ala Glu Asn Ile Glu Ala Ile Lys Asp Ala
610 615 620

ACA AAG CAG ATG ATG GTC ATC GTT GAA GAT CAA ATC AAA CAC AGT CTC 2395
Thr Lys Gln Met Met Val Ile Val Glu Asp Gln Ile Lys His Ser Leu
625 630 635

GGT AAT GCT AAC TAC GAC CGG GTC ATT GAA GCG CTG GGC ACG ATG CGT 2443
Gly Asn Ala Asn Tyr Asp Arg Val Ile Glu Ala Leu Gly Thr Met Arg
640 645 650

GAC GAG TTG GTA TCT TAC GAA GAG CCT GCC TCC TAC AAT GAC TTC CTG 2491
Asp Glu Leu Val Ser Tyr Glu Glu Pro Ala Ser Tyr Asn Asp Phe Leu

655 660 665

GGC CAG CTC AAG GAT AAG TTA CTG CAG GAG AAG CTT GGA GGA GAC CGA 2539
Gly Gln Leu Lys Asp Lys Leu Leu Gln Glu Lys Leu Gly Gly Asp Arg
670 675 680 685

CAA GAG CTG TGG TGG CTT GTT CGA CGA AAC AAG CTG GGA CTT GTC ACT 2587
Gln Glu Leu Trp Trp Leu Val Arg Arg Asn Lys Leu Gly Leu Val Thr
690 695 700

CAG CGC GAG TCG GAT CAA TCT AGG GTT ACC GAT ACG GAA GCC AAA GAA 2635
Gln Arg Glu Ser Asp Gln Ser Arg Val Thr Asp Thr Glu Ala Lys Glu
705 710 715

GTA AGT CTC ACT AAG ATG AAG GAG TGA 2662
Val Ser Leu Thr Lys Met Lys Glu Sto
720 725

## Claims

1. A method for the deletion of a large chromosomal region comprising transforming a transformant having an increased frequency of homologous recombination due to suppression of a *Ku gene,* which is a mitosporic filamentous fungus belonging to *Trichocomaceae* with a vector for the deletion of a chromosomal region, which comprises a pair of homologous region arms having a nucleotide sequence that is homologous with a fragment at either end of said chromosomal region, and deleting the chromosomal region by means of homologous recombination between the resulting transformant and said vector.

2. The method according to Claim 1, wherein the transformant having an increased frequency of homologous recombination is *Aspergillus sojae* ASKUPTR8 strain (wh, *ΔpyrG* Ku70::ptrA) (FERM BP-10453).

3. The method according to Claim 1, wherein the transformant having an increased frequency of homologous recombination is *Aspergillus oryzae* RkuN16ptr1 strain.

4. The method according to Claim 1, wherein a length of the homologous region arms is 1-3 kb.

5. The method according to any one of Claim 4, wherein the homologous region arm has a nucleotide sequence of a gene or a part thereof that is located in the most marginal part at either end of the chromosomal region to be deleted.

6. The method according to Claim 1, wherein a selective marker gene is comprised between the pair of the homologous region arms of the vector for the deletion of a chromosomal region.

7. The method according to Claim 6, wherein the selective marker gene is selected from pyrG, sC or niaD.

8. The method according to Claim 6, wherein a length of the chromosomal region to be deleted is 15 - 200kb.

9. The method according to Claim 8, wherein the chromosomal region to be deleted constitutes a cluster of biosynthesis genes encoding a toxic substance.

10. The method according to Claim 8, wherein the toxic substance is Aflatoxin.

11. A transformant produced by the method according Claim 1, which has the deletion of a part of chromosomal region.
